# EUROPEAN PATENT APPLICATION

(11) **EP 2 615 081 A1**
(43) Date of publication of application: **17.07.2013**
(21) Application number: 11823566.2
(22) Date of filing: 06.09.2011
(51) Int. Cl.: C07C 231/02, C07C 235/16, C07D 235/12, C07B 61/00

(54) **PROCESS FOR PREPARING BENZOIC ACID ESTERS**

(30) Priority: 07.09.2010 JP 2010200092
(71) Applicant: Daiichi Sankyo Company, Limited, Tokyo 103-8426 (JP)
(72) Inventor: WAKAYAMA, Masakazu, Kanagawa 254-0014 (JP); SAITO, Ayako, Kanagawa 254-0014 (JP); KAJINO, Hisaki, Kanagawa 254-0014 (JP)
(74) Representative: Nieuwenhuys, William Francis
(86) International application number: PCT/JP2011/070257
(87) International publication number: WO 2012/033091

(57) **Abstract**

There is provided a more industrially advantageous process for preparing novel pyridine derivatives expected to be used as medicines. A process for preparing 3-(6-hydroxy-1-methyl-1H-benzimidazol-2-yl)benzoic acid esters as intermediates with high quality, in short steps and in a high yield, as well as novel benzoic acid esters as their precursors and a process for preparing the same.

## Description

### Technical Field

The present invention relates to a synthetic intermediate of a medicament, in particular, a novel pyridine derivative, which has a glucose lowering effect or treats and/or prevents the onset of a disorder of glucose or lipid metabolism or a disease mediated by peroxisome proliferator-activated receptor (PPAR) γ, as well as a process for preparing the same.

### Background Art

In recent years, the number of patients with metabolic syndrome such as type II diabetes, hyperinsulinemia, dyslipidemia, adiposity, hypertension or atherosclerotic disease has been increasing around the world due to reasons such as changes in lifestyles. Patients with metabolic syndrome have a several-fold increased risk of coronary artery disease, cerebral infarction and cerebral hemorrhage and are further affected with chronic complications such as nephropathy, neuropathy and retinopathy. The increase in the number of patients with complications has been a major cause of rising medical costs (Non-Patent Document 1).

Recent researches have shown that ligands acting on PPARγ are useful for the prevention or improvement of a pathology called metabolic syndrome such as type II diabetes, hyperinsulinemia, dyslipidemia, adiposity, hypertension, atherosclerotic disease or insulin resistance (Non-Patent Document 2). Ligands acting on PPARγ inhibit the production of inflammatory cytokines (Non-Patent Documents 3 and 4) and induce apoptosis to inhibit the growth of cancer cells (Non-Patent Document 5). Therefore, the ligands are also useful for the prevention or improvement of inflammatory disease or cancer. Specific examples of the ligands activating PPARγ include pioglitazone (Non-Patent Document 6) and rosiglitazone (Non-Patent Document 7) classified into thiazolidinedione drugs already medically used in the treatment of type II diabetes. These thiazolidinedione drugs have side effects such as fluid retention, body weight increase and increased risks for heart disease. Therefore, safer pharmaceuticals have been desired to be developed (Patent Document 1). Many researchers have now been researching and developing pharmaceuticals with an aim to prevent or improve insulin resistance, diseases caused by inflammation or the like, or metabolic syndrome through researches of ligands activating or inhibiting PPARα, PPARγ or PPARδ (Non-Patent Document 8).

Patent Document 2 describes compounds having an alkoxy group, a (substituted) phenyloxy group, a pyridyloxy group or the like bonded to the 6-position of a benzimidazole group as derivatives having the same skeleton as in the compounds synthesized in the present application, and use of those compounds as therapeutic agents for diabetes, hyperglycemia or the like. However, in the synthetic examples in this document, the sole pyridyloxy group at the 6-position of the benzimidazole group is an unsubstituted 3-pyridyloxy group. On the other hand, in the compounds synthesized in the present application, a pyridyloxy group having 1 to 3 substituent(s) is bonded to the 6-position of a benzimidazole group.

### Citation List

### Patent Documents

Patent Document 1: WO 2004/014308
Patent Document 2: WO 2008/126732

### Non-patent Documents

Non-Patent Document 1: Annual Reports in Medicinal Chemistry, 39, 41-56 (2004)
Non-Patent Document 2: Annual Reviews of Medicine, 53, 409-435 (2002)
Non-Patent Document 3: Nature, 391, 79-82 (1998)
Non-Patent Document 4: Nature, 391, 82-86 (1998)
Non-Patent Document 5: Biochemical and Biophysical Research Communications, 270, 400-405 (2000)
Non-Patent Document 6: CHEMICAL & PHARMACEUTICAL BULLETIN, 39, 1440-1445 (1991)
Non-Patent Document 7: Bioorganic and Medicinal Chemistry Letter, 4, 1181-1184 (1994)
Non-Patent Document 8: Annual Reports in Medicinal Chemistry, 38, 71-80 (2003)

### Summary of the Invention

### Problems to be Solved by the Invention

Pyridine derivatives having a specific chemical structure are useful as therapeutic agents or prophylactic agents for metabolic syndrome, specifically, diseases such as diabetes (especially type II diabetes), hyperglycemia, hyperlipidemia, adiposity, impaired glucose tolerance (IGT), insulin resistance, impaired fasting glucose (IFG), hypertension, fatty liver, nonalcoholic steatohepatitis (NASH), diabetic complications (such as retinopathy, nephropathy or neuropathy), arteriosclerosis, gestational diabetes mellitus (GDM) or polycystic ovary syndrome (PCOS), inflammatory disease (such as osteoarthritis, pain or inflammatory enteritis), acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease (such as systemic lupus erythematosus, chronic rheumatism, Sjogren's syndrome, systemic sclerosis, mixed connective tissue disease, Hashimoto's disease, Crohn's disease, ulcerative colitis, idiopathic Addison's disease, male sterility, Goodpasture's syndrome, rapidly progressive glomerulonephritis, myasthenia gravis, polymyositis, multiple sclerosis, autoimmune hemolytic anemia, idiopathic thrombocytopenic purpura, Behcet's disease or CREST syndrome), pancreatitis, cachexia, cancer (such as gastric cancer, lung cancer, breast cancer, colon cancer, prostate cancer, pancreatic cancer or liver cancer), leukemia, sarcoma (such as liposarcoma), osteoporosis, involutional osteoporosis, neurodegenerative disease, Alzheimer's disease, hyperuricemia, dry eyes, or the like and are expected to be used as medicines. Accordingly, it is industrially significant to produce these derivatives with high quality, in a few steps, by a simpler operation and in a higher yield.

As a result of extensive studies for a more industrially advantageous process for preparing novel pyridine derivatives expected to be used as medicaments, the present inventors have found a process for preparing 3-(6-hydroxy-1-methyl-1H-benzimidazol-2-yl)benzoic acid esters as their synthetic intermediates with high quality, in short steps and in a high yield, as well as novel benzoic acid esters as their precursors and a process for preparing the same. This finding has led to the completion of the present invention.

### Means for Solving the Problems

The present invention relates to:
(1) A process for preparing a compound represented by general formula (3):

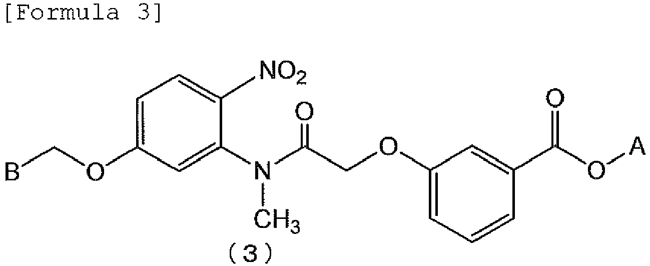

[wherein
A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group; and B represents a C₆-C₁₀ aryl group which may be substituted with a C₁-C₆ alkyl group or a C₁-C₆ alkoxy group], the process comprising reacting a compound represented by general formula (1):

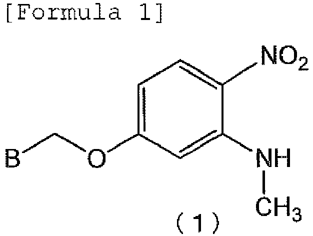

[wherein B is as defined above] with a compound represented by general formula (2):

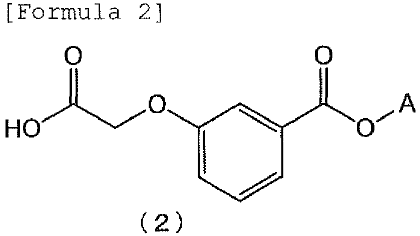

[wherein A is as defined above] in a solvent.

Preferred embodiments of the present invention include:
(2) The preparation process according to (1), wherein A is a methyl group;

(3) The preparation process according to (1) or (2), wherein B is a phenyl group;

(4) The preparation process according to any one of (1) to (3), wherein the solvent is a halogenated hydrocarbon, a nitrile, an ether or a mixed solvent thereof;

(5) The preparation process according to any one of (1) to (3), wherein the solvent is tetrahydrofuran;

(6) The preparation process according to any one of (1) to (3), wherein the solvent is a halogenated hydrocarbon, a nitrile, an ether, an amide, a carboxylate or a mixed solvent thereof;

(7) The preparation process according to any one of (1) to (3), wherein the solvent is N,N-dimethylacetamide;

(8) The preparation process according to any one of (1) to (7) in the presence of a halogenating agent;

(9) The preparation process according to (8), wherein the halogenating agent is thionyl chloride, oxalyl chloride or phosphorus pentachloride;

(10) The preparation process according to (8), wherein the halogenating agent is thionyl chloride;

(11) The preparation process according to any one of (8) to (10), wherein the compound represented by the general formula (1) and the compound represented by the general formula (2) are previously mixed and the halogenating agent is added thereto;

(12) The preparation process according to any one of (1) to (10) in the presence of a base;

(13) The preparation process according to (12), wherein the base is an alkali metal hydride;

(14) The preparation process according to (12), wherein the base is sodium hydride;

(15) The preparation process according to any one of (1) to (14) in the presence of a catalyst; and

(16) The preparation process according to (15), wherein the catalyst is N,N-dimethylformamide.

The present invention also relates to:
(17) A process for preparing a compound represented by general formula (4):

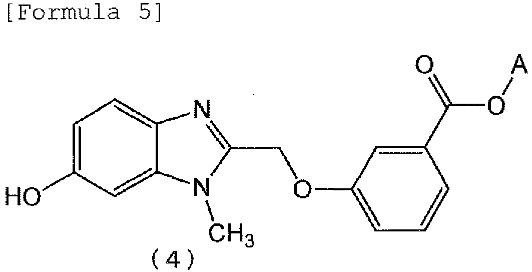

[wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group], the process comprising reacting a compound represented by general formula (3):

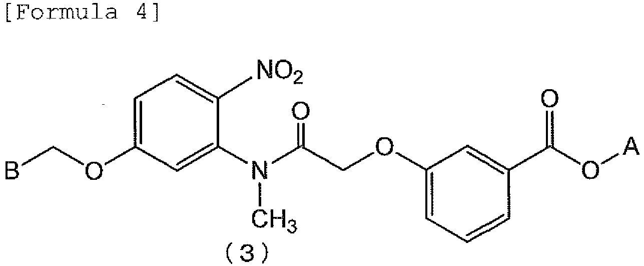

[wherein A is as defined above; and B represents a C₆-C₁₀ aryl group which may be substituted with a C₁-C₆ alkyl group or a C₁-C₆ alkoxy group] with hydrogen in a solvent in the presence of a catalyst.

Preferred embodiments of the present invention include:
(18) The preparation process according to (17), wherein A is a methyl group;

(19) The preparation process according to (17) or (18), wherein B is a phenyl group;

(20) The preparation process according to any one of (17) to (19), wherein the solvent is an alcohol, an amide or a mixed solvent thereof;

(21) The preparation process according to any one of (17) to (19), wherein the solvent is N,N-dimethylacetamide;

(22) The preparation process according to any one of (17) to (21), wherein the catalyst is a palladium-carbon catalyst or a platinum-carbon catalyst; and

(23) The preparation process according to any one of (17) to (21), wherein the catalyst is a palladium-carbon catalyst.

The present invention also relates to:
(24) A process for preparing a compound represented by general formula (5):

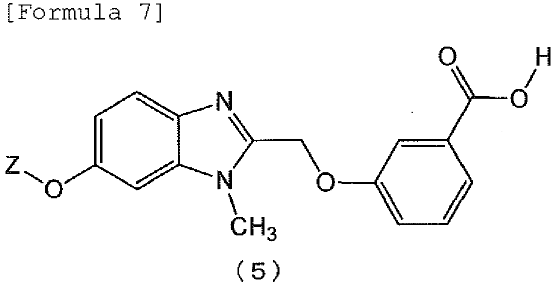

[wherein Z represents a pyridyl group independently substituted with 1 to 3 halogen atom(s), C₁-C₆ alkyl group(s) and/or C₁-C₆ alkoxy group(s)] using a compound produced in the following Step I and represented by general formula (3):

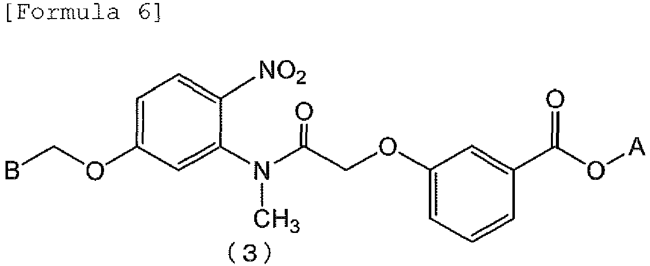

[wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group; and B represents a C₆-C₁₀ aryl group which may be substituted with a C₁-C₆ alkyl group or a C₁-C₆ alkoxy group]
{wherein Step I is a step of preparing the compound represented by the general formula (3), the step comprising reacting a compound represented by general formula (1):

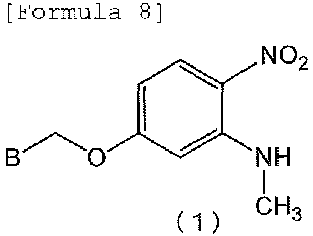

[wherein B is as defined above] with a compound represented by general formula (2):

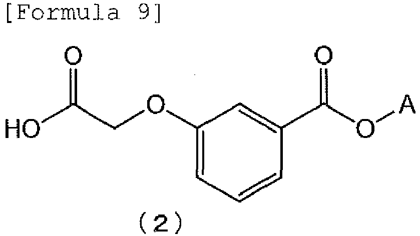

[wherein A is as defined above] in a solvent}.

Preferred embodiments of the present invention include:
(25) The preparation process according to (24), wherein A is a methyl group;

(26) The preparation process according to (24) or (25), wherein B is a phenyl group;

(27) The preparation process according to any one of (24) to (26), wherein the solvent is a halogenated hydrocarbon, a nitrile, an ether or a mixed solvent thereof;

(28) The preparation process according to any one of (24) to (26), wherein the solvent is tetrahydrofuran;

(29) The preparation process according to any one of (24) to (26), wherein the solvent is a halogenated hydrocarbon, a nitrile, an ether, an amide, a carboxylate or a mixed solvent thereof;

(30) The preparation process according to any one of (24) to (26), wherein the solvent is N,N-dimethylacetamide;

(31) The preparation process according to any one of (24) to (30) in the presence of a halogenating agent;

(32) The preparation process according to (31), wherein the halogenating agent is thionyl chloride, oxalyl chloride or phosphorus pentachloride;

(33) The preparation process according to (31), wherein the halogenating agent is thionyl chloride;

(34) The preparation process according to any one of (31) to (33), wherein the compound represented by the general formula (1) and the compound represented by the general formula (2) are previously mixed and the halogenating agent is added thereto;

(35) The preparation process according to any one of (24) to (33) in the presence of a base;

(36) The preparation process according to (35), wherein the base is an alkali metal hydride;

(37) The preparation process according to (35), wherein the base is sodium hydride;

(38) The preparation process according to any one of (24) to (37) in the presence of a catalyst; and

(39) The preparation process according to (38), wherein the catalyst is N,N-dimethylformamide.

The present invention also relates to:
(40) A process for preparing a compound represented by general formula (5) :

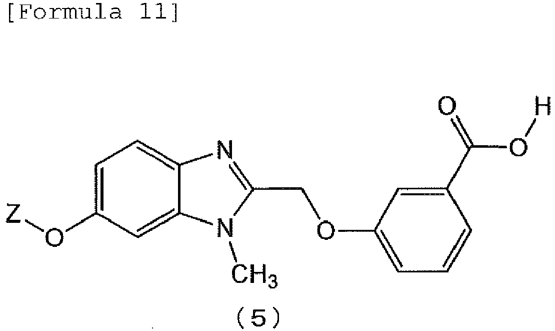

[wherein Z represents a pyridyl group independently substituted with 1 to 3 halogen atom(s), C₁-C₆ alkyl group(s) and/or C₁-C₆ alkoxy group(s)] using a compound produced in the following Step II and represented by general formula (4):

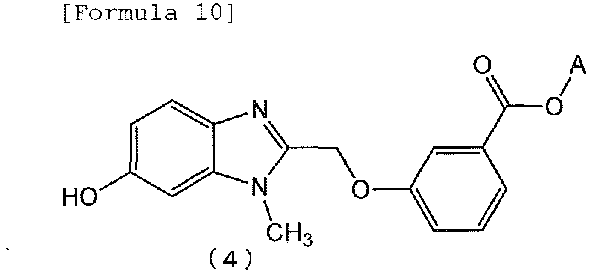

[wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group]
{wherein Step II is a step of preparing the compound represented by the general formula (4), the step comprising reacting a compound represented by general formula (3):

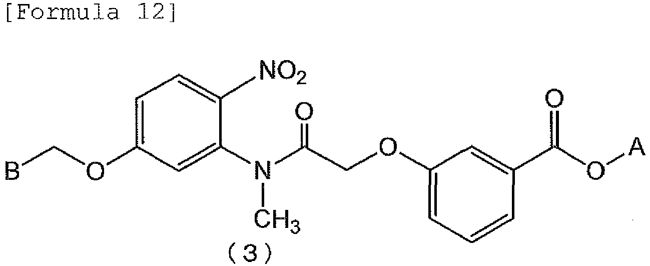

[wherein A is as defined above; and B represents a C₆-C₁₀ aryl group which may be substituted with a C₁-C₆ alkyl group or a C₁-C₆ alkoxy group] with hydrogen in a solvent in the presence of a catalyst}.

Preferred embodiments of the present invention include:
(41) The preparation process according to (40), wherein A is a methyl group;

(42) The preparation process according to (40) or (41), wherein B is a phenyl group;

(43) The preparation process according to any one of (40) to (42), wherein the solvent is an alcohol, an amide or a mixed solvent thereof;

(44) The preparation process according to any one of (40) to (42), wherein the solvent is N,N-dimethylacetamide;

(45) The preparation process according to any one of (40) to (42), wherein the catalyst is a palladium-carbon catalyst or a platinum-carbon catalyst; and

(46) The preparation process according to any one of (40) to (44), wherein the catalyst is a palladium-carbon catalyst.

The present invention relates to:
(47) A compound represented by general formula (3):

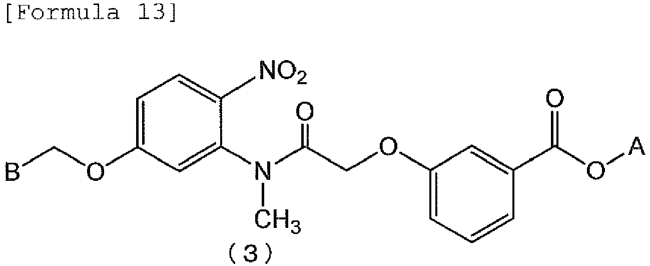

[wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group; and B represents a C₆-C₁₀ aryl group which may be substituted with a C₁-C₆ alkyl group or a C₁-C₆ alkoxy group].

Preferred embodiments of the present invention include:
(48) The compound according to (47), wherein A is a C₁-C₆ alkyl group;

(49) The compound according to (47), wherein A is a methyl group;

(50) The compound according to any one of (47) to (49), wherein B is a C₆-C₁₀ aryl group; and

(51) The compound according to any one of (47) to (49), wherein B is a phenyl group.

The "C₁-C₆ alkyl group" in the present invention is a linear or branched alkyl group having 1 to 6 carbon atom(s). Examples of such a group include a methyl group, an ethyl group, a propyl group, an isopropyl group, a butyl group, an s-butyl group, a t-butyl group, an isobutyl group, a pentyl group, a neopentyl group and a hexyl group. The group is preferably a methyl group, an ethyl group, a propyl group, an isopropyl group, a butyl group, an s-butyl group, a t-butyl group or an isobutyl group, and more preferably a methyl group.

The "C₃-C₇ cycloalkyl group" in the present invention is a cyclopropyl group, a cyclobutyl group, a cyclopentyl group, a cyclohexyl group or a cycloheptyl group, and is preferably a cyclopentyl group or a cyclohexyl group.

The "C₆-C₁₀ aryl group" in the present invention is a phenyl group, a 1-naphthyl group or a 2-naphthyl group, and is preferably a phenyl group.

The "C₁-C₆ alkoxy group" in the present invention is a linear or branched alkoxy group having 1 to 6 carbon atom(s). Examples of such a group include a methoxy group, an ethoxy group, a propoxy group, an isopropoxy group, a butoxy group, an s-butoxy group, a t-butoxy group, an isobutoxy group, a pentoxy group, a neopentoxy group and a hexyloxy group. The group is preferably a methoxy group, an ethoxy group, a propoxy group, an isopropoxy group, a butoxy group, an s-butoxy group, a t-butoxy group or a t-butoxy group, and more preferably a methoxy group or an ethoxy group.

The "C₆-C₁₀ aryl group which may be substituted with a C₁-C₆ alkyl group or a C₁-C₆ alkoxy group" in the present invention is the above-mentioned "C₆-C₁₀ aryl group", or the above-mentioned "C₆-C₁₀ aryl group" to which the above-mentioned "C₁-C₆ alkyl group" or the above-mentioned "C₁-C₆ alkoxy group" is bonded. The group is preferably a C₆-C₁₀ aryl group, and more preferably a phenyl group.

The "C₁-C₆ alkyl group which may be substituted with a C₁-C₆ alkoxy group" in the present invention is the above-mentioned "C₁-C₆ alkyl group", or the above-mentioned "C₁-C₆ alkyl group" to which the above-mentioned "C₁-C₆ alkoxy group" is bonded. The group is preferably a C₁-C₆ alkyl group, and more preferably a methyl group.

The "C₃-C₇ cycloalkyl group which may be substituted with a C₁-C₆ alkyl group or a C₁-C₆ alkoxy group" in the present invention is the above-mentioned "C₃-C₇ cycloalkyl group", or the above-mentioned "C₃-C₇ cycloalkyl group" to which the above-mentioned "C₁-C₆ alkyl group" or the above-mentioned "C₁-C₆ alkoxy group" is bonded.

The "halogen atom" in the present invention is a fluorine atom, a chlorine atom, a bromine atom or an iodine atom.

The "pyridyl group independently substituted with 1 to 3 halogen atom(s), C₁-C₆ alkyl group(s) and/or C₁-C₆ alkoxy group(s)" in the present invention is a 2-pyridyl group, a 3-pyridyl group or a 4-pyridyl group to which 1 to 3 of the above-mentioned "halogen atom(s)", the above-mentioned "C₁-C₆ alkyl group(s)" and/or the above-mentioned "C₁-C₆ alkoxy group(s)" are independently bonded.

Specific compounds represented by the general formula (3) according to the present invention include the compounds described in the following Table 1; however, the compounds represented by the general formula (3) are not limited to these compounds.

The abbreviations in the following Table 1 are as follows. Specifically,
Me represents a methyl group,
Et represents an ethyl group,
Pr represents a propyl group,
i-Pr represents an isopropyl group,
Bu represents a butyl group,
s-Bu represents an s-butyl group,
t-Bu represents a t-butyl group,
i-Bu represents an isobutyl group,
Pen represents a pentyl group,
Hex represents a hexyl group,
cy-Pen represents a cyclopentyl group,
cy-Hex represents a cyclohexyl group, and
C₆H₄-4-Me represents a 4-methylphenyl group.

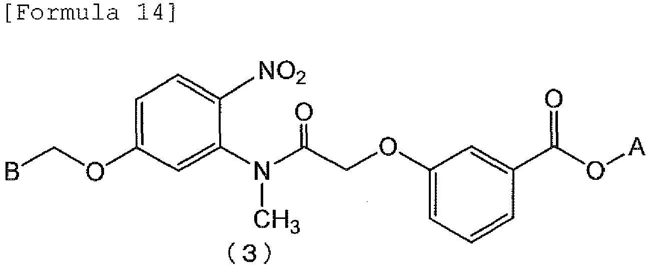

**[Table 1]**

| Compound No. | A | B |
|---|---|---|
| 1 | Me | C₆H₅ |
| 2 | Et | C₆H₅ |
| 3 | Pr | C₆H₅ |
| 4 | i-Pr | C₆H₅ |
| 5 | Bu | C₆H₅ |
| 6 | s-Bu | C₆H₅ |
| 7 | t-Bu | C₆H₅ |
| 8 | i-Bu | C₆H₅ |
| 9 | Pen | C₆H₅ |
| 10 | Hex | C₆H₅ |
| 11 | cy-Pen | C₆H₅ |
| 12 | cy-Hex | C₆H₅ |
| 13 | C₆H₅ | C₆H₅ |
| 14 | Me | C₆H₄-4-Me |
| 15 | Me | C₆H₃-3,5-Me₂ |
| 16 | Me | C₆H₄-4-OMe |
| 17 | Et | C₆H₄-4-Me |
| 18 | Et | C₆H₄-4-Et |
| 19 | Et | C₆H₄-4-OMe |
| 20 | Pr | C₆H₄-4-Me |
| 21 | Pr | C₆H₄-2-Me |
| 22 | Pr | C₆H₄-4-OMe |
| 23 | i-Pr | C₆H₄-4-Me |
| 24 | i-Pr | C₆H₄-4-O-t-Bu |

### Advantageous Effects of the Invention

The present invention can produce 3-(6-hydroxy-1-methyl-1H-benzimidazol-2-yl)benzoic acid esters as intermediates for pyridine derivatives that are useful as therapeutic agents or prophylactic agents for metabolic syndrome, specifically, diseases such as diabetes (especially type II diabetes), hyperglycemia, hyperlipidemia, adiposity, impaired glucose tolerance (IGT), insulin resistance, impaired fasting glucose (IFG), hypertension, fatty liver, nonalcoholic steatohepatitis (NASH), diabetic complications (such as retinopathy, nephropathy or neuropathy), arteriosclerosis, gestational diabetes mellitus (GDM) or polycystic ovary syndrome (PCOS), inflammatory disease (such as osteoarthritis, pain or inflammatory enteritis), acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease (such as systemic lupus erythematosus, chronic rheumatism, Sjogren's syndrome, systemic sclerosis, mixed connective tissue disease, Hashimoto's disease, Crohn's disease, ulcerative colitis, idiopathic Addison's disease, male sterility, Goodpasture's syndrome, rapidly progressive glomerulonephritis, myasthenia gravis, polymyositis, multiple sclerosis, autoimmune hemolytic anemia, idiopathic thrombocytopenic purpura, Behcet's disease or CREST syndrome), pancreatitis, cachexia, cancer (such as gastric cancer, lung cancer, breast cancer, colon cancer, prostate cancer, pancreatic cancer or liver cancer), leukemia, sarcoma (such as liposarcoma), osteoporosis, involutional osteoporosis, neurodegenerative disease, Alzheimer's disease, hyperuricemia, dry eyes, or the like and are expected to be used as medicines in a large amount, in a high yield, with high quality and in a simple manner using inexpensive reactants.

### Description of Embodiments

The process for preparing the compound represented by the general formula (3) and the target compound represented by the general formula (4) according to the present invention will be described below in detail.

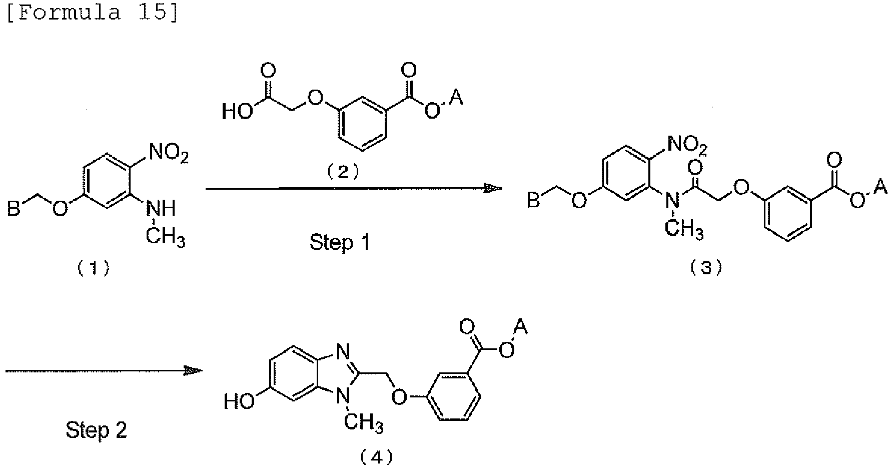

[In the formulas, A and B are as defined above].
The process of the present invention comprises Step 1 of preparing a benzoic acid ester represented by the general formula (3) by reacting an amine represented by the general formula (1) with a phenoxyacetic acid derivative represented by the general formula (2) to form an amide bond; and Step 2 of preparing a 3-(6-hydroxy-1-methyl-1H-benzimidazol-2-yl)benzoic acid ester represented by the general formula (4) by hydrogenating the benzoic acid ester represented by the general formula (3) to deprotect the (substituted) arylmethyl group and reduce the nitro group and further carrying out intramolecular dehydration condensation. The respective steps will be described below in detail.

### (Step 1)

This step is a step of preparing a benzoic acid ester (3) by reacting an amine (1) with a phenoxyacetic acid derivative (2) to form an amide bond. This step is carried out by the acid halide method, the active esterification method or the mixed acid anhydride method described in detail below.

The amine (1) that is a starting material can be easily prepared by the preparation method described in Tetrahedron Lett., 2002, 7303-7306 or Japanese Patent Laid-Open No. 2006-124375, for example. The phenoxyacetic acid derivative (2) that is the other starting material can be easily produced by the preparation method described in Pharm. Chem. J., 2001, 653 or J. Indian Chem. Soc., 1987, 34, for example.

### (Acid halide method)

The acid halide method is carried out by reacting a phenoxyacetic acid derivative (2) with a halogenating agent such as thionyl chloride or oxalyl chloride in a solvent to produce a phenoxyacetyl halide, and amidating the phenoxyacetyl halide with an amine (1) in a solvent in the presence or absence of a base.

The halogenating agent used in the halogenation is not particularly limited insofar as it can convert a carboxylic acid to an acid halide. Examples of such a halogenating agent include thionyl chloride, thionyl bromide, oxalyl chloride, phosphorous oxychloride, phosphorus trichloride and phosphorus pentachloride. Preferred examples include thionyl chloride, oxalyl chloride and phosphorus pentachloride. Particularly preferred examples include thionyl chloride.

The amount of the halogenating agent used in the halogenation is not particularly limited insofar as it is one equivalent or more based on the phenoxyacetic acid derivative (2) used, but the amount is preferably 1 to 2 equivalents, and more preferably 1 to 1.2 equivalents.

The halogenation is usually carried out in a solvent. The solvent, if used, is not particularly limited insofar as it does not inhibit the reaction. Examples of such a solvent include aliphatic hydrocarbons such as hexane, heptane, ligroin and petroleum ether; aromatic hydrocarbons such as benzene, toluene and xylene; esters such as ethyl acetate and butyl acetate; nitriles such as acetonitrile, propionitrile and benzonitrile; halogenated hydrocarbons such as dichloromethane, chloroform, 1,2-dichloroethane and carbon tetrachloride; ethers such as diethyl ether, diisopropyl ether, tetrahydrofuran, dioxane, dimethoxyethane and diethylene glycol dimethyl ether; amides such as formamide, N,N-dimethylformamide, N,N-dimethylacetamide and hexamethylphosphoric triamide; sulfoxides such as dimethyl sulfoxide; sulfolane; and mixed solvents thereof. Preferred examples include halogenated hydrocarbons, nitriles, ethers, amides, carboxylates and mixed solvents thereof. More preferred examples include halogenated hydrocarbons, nitriles, ethers and mixed solvents thereof. Still more preferred examples include ethers. Particularly preferred examples include tetrahydrofuran and N,N-dimethylacetamide. Most preferred examples include tetrahydrofuran.

In the halogenation, the reaction may proceed more rapidly by adding a catalyst. When a catalyst is added, the catalyst used is usually an amine, an amine derivative or a nitrogen-containing heterocyclic compound. When an amine is used, a tertiary amine is usually used. Examples of such an amine include trialkylamines such as trimethylamine, triethylamine, diisopropylethylamine and tributylamine; dialkylarylamines such as N,N-dimethylaniline and N,N-diethylaniline; and diarylalkylamines such as diphenylmethylamine. Examples of the amine derivative include N,N-dialkylamides such as N,N-dimethylformamide and N,N-dimethylacetamide. Examples of the nitrogen-containing heterocyclic compound include pyridine, N,N-dimethyl-4-aminopyridine, imidazole and triazole. The catalyst is preferably trimethylamine, triethylamine, diisopropylethylamine, tributylamine, N,N-dimethylaniline, N,N-dimethylformamide, N,N-dimethylacetamide, pyridine or N,N-dimethyl-4-aminopyridine, more preferably triethylamine, N,N-dimethylformamide, pyridine or N,N-dimethyl-4-aminopyridine, and particularly preferably N,N-dimethylformamide.

The amount of the catalyst used in the halogenation is not particularly limited, but is usually 0.00001 to 20 equivalents, preferably 0.0001 to 10 equivalents, and more preferably 0.001 to 5 equivalents, based on the halogenating agent used.

The reaction temperature in the halogenation varies according to the solvent, the raw material compound, the reagent and the like, but is usually -20°C to 150°C, preferably -10°C to 100°C, and more preferably -10 to 70°C.

The reaction time in the halogenation varies according to the raw material compound, the reagent, the reaction temperature and the like, but is usually 30 minutes to 80 hours, preferably 30 minutes to 48 hours, and more preferably 1 to 6 hours.

After the halogenation is completed, the amidation may be carried out after or without isolating the phenoxyacetyl halide. The amidation is preferably carried out without isolating it.

The amidation is a step of preparing a benzoic acid ester (3) and is carried out by reacting the phenoxyacetyl halide with an amine (1) in a solvent.

When a base is used in the amidation, examples of the base used include alkali metal carbonates such as lithium carbonate, sodium carbonate and potassium carbonate; alkali metal bicarbonates such as lithium bicarbonate, sodium bicarbonate and potassium bicarbonate; alkali metal hydrides such as lithium hydride, sodium hydride and potassium hydride; alkali metal hydroxides such as lithium hydroxide, sodium hydroxide and potassium hydroxide; alkali metal alkoxides such as lithium methoxide, sodium methoxide, sodium ethoxide and potassium t-butoxide; and organic amines such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]nano-5-ene, 1,4-diazabicyclo[2.2.2]octane (DABCO) and 1,8-diazabicyclo[5.4.0]-7-undecene (DBU). Preferred examples include alkali metal carbonates, alkali metal hydrides and organic amines. More preferred examples include alkali metal hydrides.

The amidation is usually carried out in a solvent. The solvent is not particularly limited insofar as it does not inhibit the reaction. Examples of such a solvent include aliphatic hydrocarbons such as hexane, heptane, ligroin and petroleum ether; aromatic hydrocarbons such as benzene, toluene and xylene; carboxylates such as ethyl acetate and butyl acetate; nitriles such as acetonitrile, propionitrile and benzonitrile; halogenated hydrocarbons such as dichloromethane, chloroform, 1,2-dichloroethane and carbon tetrachloride; ethers such as diethyl ether, diisopropyl ether, tetrahydrofuran, dioxane, dimethoxyethane and diethylene glycol dimethyl ether; amides such as formamide, N,N-dimethylformamide, N,N-dimethylacetamide and hexamethylphosphoric triamide; sulfoxides such as dimethyl sulfoxide; sulfones such as sulfolane; and mixed solvents thereof. Preferred examples include halogenated hydrocarbons, nitriles, ethers, amides and mixed solvents thereof. More preferred examples include halogenated hydrocarbons, nitriles, ethers and mixed solvents thereof. Still more preferred examples include ethers. Particularly preferred examples include tetrahydrofuran and N,N-dimethylacetamide. Most preferred examples include tetrahydrofuran.

The reaction temperature in the amidation varies according to the solvent, the raw material compound, the reagent and the like, but is usually -20°C to 150°C, and preferably -20°C to 100°C.

The reaction time in the amidation varies according to the solvent, the raw material compound, the reagent, the reaction temperature and the like, but is usually 30 minutes to 80 hours, and preferably 1 to 48 hours.

### (Active esterification method)

The active esterification method is carried out by reacting a phenoxyacetic acid derivative (2) with an active esterifying agent in a solvent in the presence or absence of a base to produce an active ester and reacting the active ester with an amine (1) to produce a benzoic acid ester (3).

Examples of the active esterifying agent used in the active esterification method include N-hydroxy compounds such as N-hydroxysuccinimide, 1-hydroxybenztriazole and N-hydroxy-5-norbornene-2,3-dicarboxylimide; disulfide compounds such as dipyridyl disulfide; carbodiimides such as dicyclohexylcarbodiimide; and condensing agents such as carbonyldiimidazole and triphenylphosphine.

The solvent used in the active esterification method is not particularly limited insofar as it does not inhibit the reaction. Examples of such a solvent include aliphatic hydrocarbons such as hexane, heptane, ligroin and petroleum ether; aromatic hydrocarbons such as benzene, toluene and xylene; carboxylates such as ethyl acetate and butyl acetate; nitriles such as acetonitrile, propionitrile and benzonitrile; halogenated hydrocarbons such as dichloromethane, chloroform, 1,2-dichloroethane and carbon tetrachloride; ethers such as diethyl ether, diisopropyl ether, tetrahydrofuran, dioxane, dimethoxyethane and diethylene glycol dimethyl ether; amides such as formamide, N,N-dimethylformamide, N,N-dimethylacetamide and hexamethylphosphoric triamide; sulfoxides such as dimethyl sulfoxide; sulfolane; and mixed solvents thereof. Preferred examples include halogenated hydrocarbons, nitriles, ethers, amides and mixed solvents thereof. More preferred examples include acetonitrile, dichloromethane, chloroform, tetrahydrofuran, dioxane, N,N-dimethylformamide and mixed solvents thereof. Particularly preferred examples include tetrahydrofuran, dioxane and acetonitrile.

When a base is used in the active esterification method, the same base as used in the above acid halide method can be used.

The reaction temperature in the active esterification method varies according to the raw material compound, the reagent and the like, but is usually -70°C to 150°C, and preferably -20°C to 100°C.

The reaction time in the active esterification method varies according to the raw material compound, the reagent, the reaction temperature and the like, but is usually 10 minutes to 80 hours, and preferably 30 minutes to 12 hours.

### (Mixed acid anhydride method)

The mixed acid anhydride method is carried out by reacting a phenoxyacetic acid derivative (2) with a mixed acid anhydride forming agent in a solvent in the presence or absence of a base to produce a mixed acid anhydride and reacting the mixed acid anhydride with an amine (1) to produce a benzoic acid ester (3).

Examples of the mixed acid anhydride forming agent used in the mixed acid anhydride method include alkanoyl halides such as acetyl chloride and pivaloyl chloride; chlorocarbonates such as methyl chlorocarbonate, ethyl chlorocarbonate and phenyl chlorocarbonate; and cyanophosphonates such as diethyl cyanophosphonate and diphenyl cyanophosphonate.

The solvent used in the mixed acid anhydride method is not particularly limited insofar as it does not inhibit the reaction. Examples of such a solvent include aliphatic hydrocarbons such as hexane, heptane, ligroin and petroleum ether; aromatic hydrocarbons such as benzene, toluene and xylene; carboxylates such as ethyl acetate and butyl acetate; nitriles such as acetonitrile, propionitrile and benzonitrile; halogenated hydrocarbons such as dichloromethane, chloroform, 1,2-dichloroethane and carbon tetrachloride; ethers such as diethyl ether, diisopropyl ether, tetrahydrofuran, dioxane, dimethoxyethane and diethylene glycol dimethyl ether; amides such as formamide, N,N-dimethylformamide, N,N-dimethylacetamide and hexamethylphosphoric triamide; sulfoxides such as dimethyl sulfoxide; sulfolane; and mixed solvents thereof. Preferred examples include halogenated hydrocarbons, nitriles, ethers, amides and mixed solvents thereof. More preferred examples include acetonitrile, dichloromethane, chloroform, tetrahydrofuran, dioxane, N,N-dimethylformamide and mixed solvents thereof. Particularly preferred examples include tetrahydrofuran, dioxane and acetonitrile.

When a base is used in the mixed acid anhydride method, the same base as used in the above acid halide method can be used.

The reaction temperature in the mixed acid anhydride method varies according to the raw material compound, the reagent and the like, but is usually -70°C to 150°C, and preferably -20°C to 100°C.

The reaction time in the mixed acid anhydride method varies according to the raw material compound, the reagent, the reaction temperature and the like, but is usually 10 minutes to 80 hours, and preferably 30 minutes to 12 hours.

The acid halide method, the active esterification method and the mixed acid anhydride method may also be performed by a method of previously mixing a phenoxyacetyl compound (2) with an amine (1) and adding a halogenating agent, an active esterifying agent or a mixed acid anhydride forming agent thereto, without carrying out halogenation, active esterification or mixed acid anhydride formation and amidation stepwise.

After the reaction is completed in the acid halide method, the active esterification method and the mixed acid anhydride method, the benzoic acid ester (3) is separated from the reaction mixture either by spontaneous crystallization or by an operation such as extraction or spontaneous crystallization following common post-treatment and optionally neutralization treatment. The resulting benzoic acid ester (3) may be used in the next step as is, or may be used after purifying it by a common purification method such as recrystallization, reprecipitation or chromatography if necessary.

### (Step 2)

This step is a step of preparing a 3-(6-hydroxy-1-methyl-1H-benzimidazol-2-yl)benzoic acid ester (4) by hydrogenating the benzoic acid ester (3) to deprotect the (substituted) arylmethyl group and reduce the nitro group and further carrying out intramolecular dehydration condensation. This step is considered to proceed through compounds represented by the following general formula(s) (6) and/or (7) and/or (8) and/or (9).

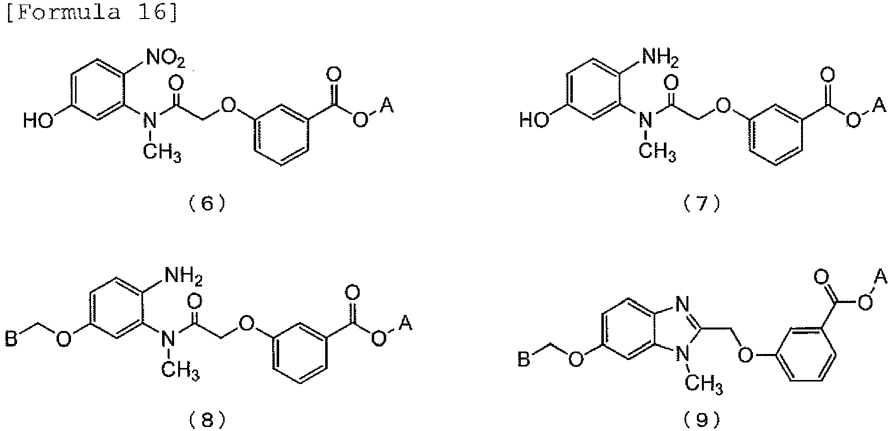

[In the formulas, A and B are as defined above].
The (substituted) arylmethyl group is deprotected and the nitro group is reduced usually by catalytic hydrogenation.

The catalyst used in this step is not particularly limited insofar as it is commonly used in catalytic hydrogenation. Examples of such a catalyst used include a palladium-carbon catalyst, a platinum-carbon catalyst, Raney nickel and a Wilkinson complex. The catalyst is preferably a palladium-carbon catalyst or a platinum-carbon catalyst.

The amount of the catalyst used in this step is not particularly limited, but is usually 0.00001 to 1 equivalent, preferably 0.0001 to 0.5 equivalent, and more preferably 0.001 to 0.3 equivalent, based on the benzoic acid ester (3).

The hydrogen pressure in this step is not particularly limited, but is usually 1 to 20 atm, and preferably 1 to 10 atm.

This step is usually carried out in a solvent. The solvent is not particularly limited insofar as it dissolves the benzoic acid ester (3) to some extent and does not inhibit the reaction. Examples of such a solvent include aliphatic hydrocarbons such as hexane, heptane, ligroin, petroleum ether, cyclohexane and methylcyclohexane; aromatic hydrocarbons such as benzene, toluene and xylene; carboxylic acids such as acetic acid; carboxylates such as ethyl acetate and butyl acetate; nitriles such as acetonitrile, propionitrile and benzonitrile; ethers such as diethyl ether, diisopropyl ether, tetrahydrofuran, dioxane, dimethoxyethane and diethylene glycol dimethyl ether; amides such as formamide, N,N-dimethylformamide, N,N-dimethylacetamide and hexamethylphosphoric triamide; and mixed solvents thereof. Preferred examples include alcohols, carboxylic acids, carboxylates, nitriles, ethers, amides and mixed solvents thereof. More preferred examples include alcohols, amides and mixed solvents thereof. Still more preferred examples include methanol, acetonitrile, tetrahydrofuran, N,N-dimethylacetamide and mixed solvents thereof. Particularly preferred examples include methanol and N,N-dimethylacetamide. Especially preferred examples include N,N-dimethylacetamide.

In this step, the reaction may be allowed to proceed by adding an acid. An acid may also be added to obtain a salt of the 3-(6-hydroxy-1-methyl-1H-benzimidazol-2-yl)benzoic acid ester (4) as is.

When an acid is added in this step, the acid added is not particularly limited. Examples of the acid used include organic acids such as hydroxyacetic acid, oxalic acid and citric acid; and hydrohalic acids such as hydrochloric acid and bromic acid. Hydrochloric acid is preferred.

When an acid is added in this step, the amount of the acid added is not particularly limited, but is usually 0.01 to 100 equivalents, and preferably 1 to 10 equivalents, based on the benzoic acid ester (3).

The reaction temperature in this step is not particularly limited, but is usually 0°C to 150°C, and preferably room temperature to 100°C.

After the reaction is completed in this step, the product is made acidic, neutral or basic depending on the properties of the product following post-treatment such as removal of the catalyst, and is then subjected to isolation operation. After the isolation, the product may be used as is, or may be purified by a common purification method such as recrystallization or chromatography if necessary.

### Examples

The present invention will be described in more detail below by way of examples.

### (Example 1)

### Methyl 3-(2-{[5-(benzyloxy)-2-nitrophenyl](methyl)amino}-2-oxoethoxy)benzoate

Thionyl chloride (12.16 g, 0.102 mol) and N,N-dimethylformamide (0.41 g, 0.56 mmol) were added to a suspension of 5-(benzyloxy)-N-methyl-2-nitroaniline (Tetrahedron Lett., 2002, 7303-7306) (20.00 g, 77.4 mmol) and [3-(methoxycarbonyl)phenoxy]acetic acid (19.53 g, 92.9 mmol) in tetrahydrofuran (100 mL) in a nitrogen stream at 10 to 30°C, and the mixture was stirred at 20 to 30°C for 24 hours. After stirring the reaction solution at 5 to 10°C for two hours, the precipitated crystals were separated by filtration, washed with isopropyl acetate (100 mL) and then dried under reduced pressure to obtain the title compound (32.12 g, 71.29 mmol). Yield: 92%.
¹H-NMR (mixture of rotamers, 500 MHz, DMSO-d₆): δ ppm: 3.07, 3.28 (3H, s, s), 3.80, 3.81 (3H, s, s), 4.44, 4.57, 5.08 (2H, d, J = 14.9 Hz, d, J = 14.9 Hz, s), 5.22, 5.24 (2H, s, s), 7.00-7.53 (11H, m), 8.01, 8.20 (1H, d, J = 9.2 Hz, d, J = 9.2 Hz). ;
Anal. Calcd. for C₂₄H₂₂N₂O₇: C, 63.99; H, 4.92; N, 6.22. Found C, 63.76; H, 4.93; N, 6.27.

### (Example 2)

### Methyl 3-(2-{[5-(benzyloxy)-2-nitrophenyl](methyl)amino}-2-oxoethoxy)benzoate

N,N-Dimethylformamide (2 µL, 0.03 mmol) and thionyl chloride (37 µL, 0.51 mmol) were added to a solution of [3-(methoxycarbonyl)phenoxy]acetic acid (98 mg, 0.47 mmol) in tetrahydrofuran (1 mL) at 20 to 30°C, and the mixture was stirred at 50°C for three hours. The reaction solution was concentrated under reduced pressure to provide an oil. Tetrahydrofuran (3 mL) was added to the oil, and the mixture was concentrated under reduced pressure to provide an oil. The same operation was then performed again to obtain methyl 3-(chlorocarbonylmethoxy)benzoate as an oil.

A solution of methyl 3-(chlorocarbonylmethoxy)benzoate in tetrahydrofuran (1 mL) was added to a suspension of 100 mg of 5-(benzyloxy)-N-methyl-2-nitroaniline (0.39 mmol) in tetrahydrofuran (1 mL) in a nitrogen stream at 20 to 30°C, and the mixture was stirred at the same temperature for 15 hours. The precipitated crystals were separated by filtration, washed with diethyl ether (100 mL) and then dried under reduced pressure to obtain the title compound (140 mg, 0.31 mmol). Yield: 80%.

### (Example 3)

### Methyl 3-(2-{[5-(benzyloxy)-2-nitrophenyl](methyl)amino}-2-oxoethoxy)benzoate

N,N-Dimethylformamide (2 µL, 0.03 mmol) and thionyl chloride (37 µL, 0.51 mmol) were added to a solution of [3-(methoxycarbonyl)phenoxy]acetic acid (98 mg, 0.47 mmol) in tetrahydrofuran (1 mL) at 20 to 30°C, and the mixture was stirred at 50°C for three hours. The reaction solution was concentrated under reduced pressure to provide an oil. Tetrahydrofuran (3 mL) was added to the oil, and the mixture was concentrated under reduced pressure to provide an oil. The same operation was then performed again to obtain methyl 3-(chlorocarbonylmethoxy)benzoate as an oil.

Sodium hydride (content: 55%, 17 mg, 0.39 mmol) was added to a suspension of 5-(benzyloxy)-N-methyl-2-nitroaniline (100 mg, 0.39 mmol) in tetrahydrofuran (1 mL) in a nitrogen stream at 0 to 5°C, followed by stirring for five minutes. A solution of methyl 3-(chlorocarbonylmethoxy)benzoate in tetrahydrofuran (1 mL) was added, and the mixture was stirred at 20 to 30°C for 12 hours. The reaction solution was stirred at 0 to 5°C for 10 minutes, and the precipitated crystals were then separated by filtration to obtain wet crystals of methyl 3-(2-{[5-(benzyloxy)-2-nitrophenyl](methyl)amino}-2-oxoethoxy)benzoate. This was dissolved in an appropriate amount of dimethoxyethane. After filtration, the filtrate was concentrated under reduced pressure. The resulting crystals were separated by filtration, washed with diethyl ether (1 mL) and then dried under reduced pressure to obtain the title compound (136 mg, 0.30 mmol). Yield: 78%.

### (Example 4)

### Methyl 3-[(6-hydroxy-1-methyl-1H-benzimidazol-2-yl)methoxy]benzoate

5% palladium-carbon (53% wet, 3.58 g, 0.95 mmol) was added to a solution of methyl 3-(2-{[5-(benzyloxy)-2-nitrophenyl](methyl)amino}-2-oxoethoxy)benzoate obtained in Example 1 (32.00 g, 71.04 mmol) in N,N-dimethylacetamide (224 mL). The mixture was stirred at 20 to 30°C for 0.5 hour and then at 65 to 75°C for four hours under 0.3 MPa hydrogen pressure. The palladium-carbon was separated by filtration, and washed with N,N-dimethylacetamide (32 mL). Water (48 mL) was added to the filtrate at 30 to 40°C, and water (80 mL) was then added dropwise thereto at 35 to 45°C. The mixture was stirred at the same temperature for 0.5 hour. Water (160 mL) was further added dropwise at the same temperature, and the mixture was stirred at the same temperature for 0.5 hour, at 20 to 30°C for two hours, and at 5 to 15°C for one hour. The precipitated crystals were separated by filtration, washed with water (110 mL) and then dried under reduced pressure to obtain the title compound (21.16 g, 67.70 mmol). Yield: 95%.
¹H-NMR (400 MHz, DMSO-d₆): δ ppm: 3.74 (3H, s), 3.85 (3H, s), 5.40 (2H, s), 6.71 (1H, dd, J = 2.4, 8.6 Hz), 6.83 (1H, d, J = 2.0 Hz), 7.40-7.43 (2H, m), 7.47 (1H, t, J = 7.4 Hz), 7.58 (1H, dt, J = 1.6, 7.8 Hz), 7.63-7.64 (1H, m), 9.36 (1H, s).

### (Example 5)

### Methyl 3-(2-{[5-(benzyloxy)-2-nitrophenyl](methyl)amino}-2-oxoethoxy)benzoate

Thionyl chloride (0.37 mL, 5.07 mmol) and N,N-dimethylformamide (22 µL, 0.28 mmol) were added to a suspension of 5-(benzyloxy)-N-methyl-2-nitroaniline (1.00 g, 3.87 mmol) and [3-(methoxycarbonyl)phenoxy]acetic acid (0.98 g, 4.66 mmol) in N,N-dimethylacetamide (5 mL) in a nitrogen stream at 10 to 30°C, and the mixture was stirred at 20 to 30°C for three hours. After cooling the reaction solution to 0 to 5°C, water (5 mL) was added dropwise, and the mixture was stirred at the same temperature for 15 hours. The precipitated crystals were separated by filtration, sufficiently washed with isopropyl acetate and water and then dried under reduced pressure to obtain the title compound (1.66 g, 3.69 mmol). Yield: 95%.

### (Reference Example 1)

### 3-{{6-[(3-Chloropyridin-2-yl)oxy]-1-methyl-1H-benzimidazol-2-yl}methoxy)benzoic acid

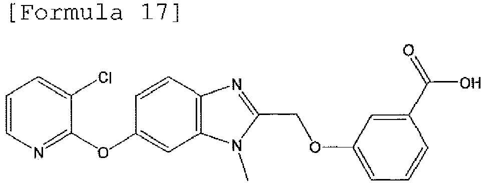

### (1a) Methyl 3-({6-[(3-chloropyridin-2-yl)oxy]-1-methyl-1H-benzimidazol-2-yl}methoxy)benzoate

A solution of methyl 3-[(6-hydroxy-1-methyl-1H-benzimidazol-2-yl)methoxy]benzoate (3.12 g, 10 mmol), 3-chloro-2-fluoropyridine (1.45 g, 11 mmol), copper iodide (0.19 g, 1.0 mmol), 1,10-phenanthroline (0.18 g, 1.0 mmol) and cesium carbonate (9.77 g, 30 mmol) in DMF (50 mL) was stirred in a nitrogen atmosphere at 80°C for two hours. After leaving to cool, a saturated ammonium chloride aqueous solution (200 mL) was added to the reaction mixture, followed by extraction with ethyl acetate (200 mL). Then, the organic layer was washed with water (200 mL) twice and dried over anhydrous sodium sulfate. After concentration under reduced pressure, the residue was purified by silica gel chromatography (methylene chloride/methanol, 95:5) to obtain the title compound (2.46 g, 58%) as a white solid.
¹H-NMR (400 MHz, CDCl₃) : δ ppm: 3.87 (3H, s), 3.93 (3H, s), 5.42 (2H, s), 6.98 (1H, dd, J = 4.7, 7.4 Hz), 7.10
(1H, dd, J = 2.4, 9.0 Hz), 7.22 (1H, d, J = 2.0 Hz), 7.30 (1H, dd, J = 1.0, 8.6 Hz), 7.38 (1H, t, J = 7.8 Hz), 7.70 (1H, d, J = 7.4 Hz), 7.72-7.73 (1H, m), 7.79 (1H, dd, J = 1.6, 7.4 Hz), 7.80 (1H, d, J = 8.6 Hz), 8.02 (1H, dd, J = 1.6, 4.7 Hz).

### (1b) 3-({6-[(3-Chloropyridin-2-yl)oxy]-1-methyl-1H-benzimidazol-2-yl}methoxy)benzoic acid

Methyl 3-({6-[(3-chloropyridin-2-yl)oxy]-1-methyl-1H-benzimidazol-2-yl}methoxy)benzoate produced in Reference Example (1a) (2.46 g, 5.8 mmol), a 2 M sodium hydroxide aqueous solution (10 mL) and 1,4-dioxane (20 mL) were stirred at 80°C for two hours. After leaving to cool, the reaction mixture was concentrated and water (100 mL) was added. This aqueous solution was neutralized by adding 1 M hydrochloric acid and the precipitated solid was collected by filtration to obtain the title compound (1.59 g, 67%) as a white solid.
¹H-NMR (400 MHz, DMSO-d₆) : δ ppm: 3.83 (3H, s), 5.49 (2H, s), 7.01 (1H, dd, J = 2.4, 9.0 Hz), 7.15 (1H, dd, J = 5.1, 7.8 Hz), 7.37-7.40 (1H, m), 7.45 (1H, t, J = 7.4 Hz), 7.49 (1H, d, J = 2.4 Hz), 7.57 (1H, d, J = 7.8 Hz), 7.63-7.64 (1H, m), 7.67 (1H, d, J = 8.6 Hz), 8.03 (1H, dd, J = 2.0, 5.1 Hz), 8.06 (1H, dd, J = 1.6, 7.8 Hz), 13.06 (1H, brs);

Anal. Calcd for C₂₁H₁₆ClN₃O₄: C, 61.54; H, 3.94; N, 10.25. Found C, 61.40; H, 3.86; N, 10.17.

### (Reference Example 2)

3-({6-[(3-Ethylpyridin-2-yl)oxy]-1-methyl-1H-benzimidazol-2-yl}methoxy)benzoic acid

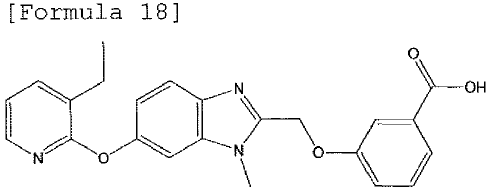

### (2a) Methyl 3-({6-[(3-bromopyridin-2-yl)oxyl-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (3.12 g, 10 mmol), 3-bromo-2-fluoropyridine (1.94 g, 11 mmol), copper iodide (0.19 g, 1.0 mmol), 1,10-phenanthroline (0.18 g, 1.0 mmol), cesium carbonate (9.77 g, 30 mmol) and DMF (50 mL) to obtain the title compound (2.19 g, 47%) as a white solid.
¹H-NMR (400 MHz, CDCl₃): δ ppm: 3.87 (3H, s), 3.93 (3H, s), 5.42 (2H, s), 6.91 (1H, dd, J = 5.1, 8.6 Hz), 7.10 (1H, dd, J = 2.7, 8.6 Hz), 7.21 (1H, d, J = 2.4 Hz), 7.30 (1H, dd, J = 0.8, 8.2 Hz), 7.38 (1H, t, J = 7.8 Hz), 7.68 (1H, d, J = 7.8 Hz), 7.72-7.73 (1H, m), 7.80 (1H, d, J = 8.6 Hz), 7.96 (1H, dd, J = 2.0, 8.6 Hz), 8.06 (1H, dd, J = 1.6, 4.7 Hz).

### (2b) Methyl 3-({6-[(3-ethylpyridin-2-yl)oxy]-1-methyl-1H-benzimidazol-2-yl}methoxy)benzoate

A solution of methyl 3-({6-[(3-bromopyridin-2-yl)oxy]-1-methyl-1H-benzimidazol-2-yl}methoxy)benzoate produced in Reference Example (2a) (1.20 g, 2.56 mmol), triethylborane (1.0 M solution in THF, 5.12 mL, 5.12 mmol), a [1,1'-bis(diphenylphosphino)-ferrocene]dichloropalladium (II)-dichloromethane mixture (0.21 g, 0.26 mmol) and potassium carbonate (0.71 g, 5.12 mmol) in DMF (10 mL) was stirred in a nitrogen atmosphere at 80°C for two days. After leaving to cool, water (50 mL) was added to the reaction mixture, followed by extraction with ethyl acetate (50 mL). Then, the organic layer was washed with water (100 mL) twice and dried over anhydrous sodium sulfate. After concentration under reduced pressure, the residue was purified by reverse phase column chromatography (acetonitrile/water, 2:1) to obtain the title compound (0.55 g, 51%) as a white solid.
¹H-NMR (400 MHz, CDCl₃) : δ ppm: 1.33 (3H, t, J = 7.4 Hz), 2.81 (2H, q, J = 7.4 Hz), 3.85 (3H, s), 3.93 (3H, s), 5.42 (2H, s), 6.95 (1H, dd, J = 5.1, 7.4 Hz), 7.05 (1H, dd, J = 2.4, 8.6 Hz), 7.16 (1H, d, J = 2.4 Hz), 7.28-7.31 (1H, m), 7.37 (1H, t, J = 8.2 Hz), 7.57 (1H, dd, J = 2.0, 7.0 Hz), 7.68-7.70 (1H, m), 7.72-7.73 (1H, m), 7.78 (1H, d, J = 8.6 Hz), 7.99 (1H, dd, J = 2.0, 4.7 Hz).

### (2c) 3-({6-[(3-Ethylpyridin-2-yl)oxy]-1-methyl-1H-benzimidazol-2-yl}methoxy)benzoic acid

The reaction and post-treatment were carried out according to Reference Example (1b) using methyl 3-({6-[(3-ethylpyridin-2-yl)oxy]-1-methyl-1H-benzimidazol-2-yl}methoxy)benzoate produced in Reference Example (2b) (0.55 g, 1.32 mmol), a 2 M sodium hydroxide aqueous solution (5 mL) and 1,4-dioxane (10 mL) to obtain the title compound (0.44 g, 89%) as a white solid. ¹H-NMR (400 MHz, DMSO-d₆) : δ ppm: 1.27 (3H, t, J = 7.8 Hz), 2.74 (2H, q, J = 7.4 Hz), 3.82 (3H, s), 5.48 (2H, s), 6.94 (1H, dd, J = 2.4, 8.2 Hz), 7.05 (1H, dd, J = 4.7, 7.0 Hz), 7.38-7.40 (2H, m), 7.45 (1H, t, J = 7.8 Hz), 7.57 (1H, d, J = 7.4 Hz), 7.62-7.64 (2H, m), 7.71 (1H, dd, J = 1.2, 7.4 Hz), 7.90 (1H, dd, J = 1.2, 4.7 Hz), 13.05 (1H, brs);
Anal. Calcd for C₂₃H₂₁N₃O₄: C, 68.47; H, 5.25; N, 10.42. Found C, 68.21; H, 5.15; N, 10.39.

### (Reference Example 3)

3-({6-[(6-Methoxy-5-methylpyridin-2-yl)oxy]-1-methyl-1H-benzimidazol-2-yl}methoxy)benzoic acid

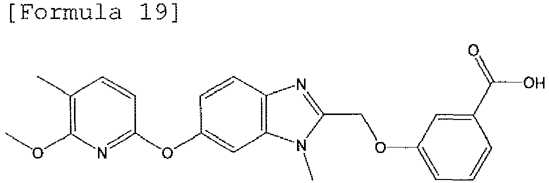

### (3a) Methyl 3-({6-[(5-bromo-6-chloropyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (1.56 g, 5.0 mmol), 3-bromo-2-chloro-6-fluoropyridine (1.16 g, 5.50 mmol), copper iodide (0.10 g, 0.50 mmol), 1,10-phenanthroline (0.09 g, 0.50 mmol), cesium carbonate (4.89 g, 15 mmol) and DMF (30 mL) to obtain the title compound (1.70 g, 68%) as a white solid.
¹H-NMR (400 MHz, DMSO-d₆) : δ ppm: 3.84 (3H, s), 3.86 (3H, s), 5.50 (2H, s), 6.98 (1H, dd, J = 0.8, 9.0 Hz), 7.05 (1H, d, J = 9.9 Hz), 7.42-7.52 (3H, m), 7.59 (1H, d, J = 8.2 Hz), 7.67-7.71 (2H, m), 8.22 (1H, dd, J = 1.6, 8.6 Hz).

### (3b) Methyl 3-({6-[(5-bromo-6-methoxypyridin-2-yl)oxy]-1-methyl-1H-benzimidazol-2-yl}methoxy)benzoate

Methyl 3-({6-[(5-bromo-6-chloropyridin-2-yl)oxy]-1-methyl-1H-benzimidazol-2-y1}methoxy)benzoate produced in Reference Example (3a) (0.74 g, 1.47 mmol), sodium methoxide (5.0 M solution in methanol, 2.94 mL, 14.7 mmol), water (10 mL) and 1,4-dioxane (20 mL) were stirred with heating under reflux for three days. After leaving to cool, the reaction mixture was concentrated and water (50 mL) was added. This aqueous solution was neutralized by adding 1 M hydrochloric acid and the precipitated solid was collected by filtration to obtain crude 3-({6-[(5-bromo-6-methoxypyridin-2-yl)oxy]-1-methyl-1H-benzimidazol-2-yl}methoxy)benzoic acid. Trimethylsilyldiazomethane (2.0 M solution in hexane) was added to a solution of the crude 3-({6-[(5-bromo-6-methoxypyridin-2-yl)oxy]-1-methyl-1H-benzimidazol-2-yl}methoxy)benzoic acid in toluene (20 mL) and methanol (10 mL) until the raw material disappeared. The reaction mixture was concentrated under reduced pressure. Then, the residue was purified by silica gel chromatography (hexane/ethyl acetate, 1:1) to obtain the title compound (0.36 g, 49%) as a white solid.
¹H-NMR (400 MHz, CDCl₃) δ ppm: 3.86 (3H, s), 3.87 (3H, s), 3.94 (3H, s), 5.42 (2H, s), 6.27 (1H, d, J = 8.2 Hz), 7.10 (1H, dd, J = 2.4, 9.0 Hz), 7.17 (1H, d, J = 2.0 Hz), 7.30 (1H, dd, J = 2.0, 8.2 Hz), 7.39 (1H, t, J = 7.8 Hz), 7.69-7.78 (4H, m).

### (3c) Methyl 3-({6-[(6-methoxy-5-methylpyridin-2-yl)oxy]-1-methyl-1H-benzimidazol-2-yl}methoxy)benzoate

The reaction and post-treatment were carried out according to Reference Example (2b) using methyl 3-({6-[(5-bromo-6-methoxypyridin-2-yl)oxy]-1-methyl-1H-benzimidazol-2-yl}methoxy)benzoate produced in Reference Example (3b) (0.36 g, 0.72 mmol), trimethylboroxine (50% solution in THF, 0.40 mL, 1.43 mmol), a [1,1'-bis(diphenylphosphino)-ferrocene]dichloropalladium (II)-dichloromethane mixture (59 mg, 0.07 mmol), potassium carbonate (0.20 g, 1.43 mmol) and DMF (10 mL) to obtain the title compound (0.26 g, 85%) as a white solid.
¹H-NMR (400 MHz, CDCl₃): δ ppm: 2.15 (3H, s), 3.85 (3H, s), 3.86 (3H, s), 3.93 (3H, s), 5.41 (2H, s), 6.20 (1H, d, J = 7.8 Hz), 7.10 (1H, dd, J = 2.0, 8.6 Hz), 7.16 (1H, d, J = 2.0 Hz), 7.30-7.32 (1H, m), 7.34 (1H, dd, J = 0.8, 7.8 Hz), 7.39 (1H, t, J = 7.8 Hz), 7.69-7.76 (3H, m).

### (3d) 3-({6-[(6-Methoxy-5-methylpyridin-2-yl)oxy]-1-methyl-1H-benzimidazol-2-yl}methoxy)benzoic acid

The reaction and post-treatment were carried out according to Reference Example (1b) using methyl 3-({6-[(6-methoxy-5-methylpyridin-2-yl)oxy]-1-methyl-1H-benzimidazol-2-yl}methoxy)benzoate produced in Reference Example (3c) (0.26 g, 0.61 mmol), a 1 M sodium hydroxide aqueous solution (20 mL) and 1,4-dioxane (40 mL) to obtain the title compound (0.22 g, 87%) as a white solid.
¹H-NMR (400 MHz, DMSO-d₆): δ ppm: 2.09 (3H, s), 3.70 (3H, s), 3.83 (3H, s), 5.47 (2H, s), 6.32 (1H, d, J = 7.8 Hz), 7.00 (1H, dd, J = 2.0, 8.6 Hz), 7.38 (1H, dd, J = 2.4, 7.8 Hz), 7.43-7.47 (2H, m), 7.53 (1H, d, J = 7.8 Hz), 7.58 (1H, d, J = 7.8 Hz), 7.64-2-7.67 (2H, m), 13.04 (1H, brs);
Anal. Calcd for C₂₃H₂₁N₃O₅·0.25H₂O: C, 65.16; H, 5.11; N, 9.91. Found C, 65.45; H, 4.98; N, 9.96.

### (Reference Example 4)

3-({6-[(5,6-Dimethylpyridin-2-yl)oxy]-1-methyl-1H-benzimidazol-2-yl}methoxy)benzoic acid

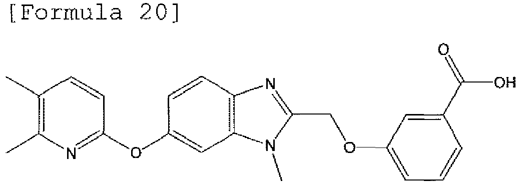

### (4a) Methyl 3-({6-[{5-bromo-6-methylpyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (3.12 g, 10 mmol), 3-bromo-6-fluoro-2-methylpyridine (2.09 g, 11 mmol), copper iodide (0.19 g, 1.0 mmol), 1,10-phenanthroline (0.18 g, 1.0 mmol), cesium carbonate (9.77 g, 30 mmol) and DMF (50 mL) to obtain the title compound (0.68 g, 14%) as a white solid.
¹H-NMR (400 MHz, CDCl₃): δ ppm: 2.54 (3H, s), 3.86 (3H, s), 3.93 (3H, s), 5.42 (2H, s), 6.53 (1H, d, J = 8.6 Hz), 7.07 (1H, dd, J = 2.4, 8.6 Hz), 7.15 (1H, d, J = 2.0 Hz), 7.31 (1H, ddd, J = 1.2, 2.7, 8.2 Hz), 7.39 (1H, t, J = 7.4 Hz), 7.69-7.73 (2H, m), 7.77 (1H, d, J = 8.6 Hz).

### (4b) Methyl 3-({6-[(5,6-dimethylpyridin-2-yl)oxy]-1-methyl-1H-benzimidazol-2-yl}methoxy)benzoate

The reaction and post-treatment were carried out according to Reference Example (2b) using methyl 3-({6-[(5-bromo-6-methylpyridin-2-yl)oxy]-1-methyl-1H-benzimidazol-2-yl}methoxy)benzoate produced in Reference Example (4a) (0.68 g, 1.41 mmol), trimethylboroxine (50% solution in THF, 0.39 mL, 1.41 mmol), a [1,1'-bis(diphenylphosphino)-ferrocene]dichloropalladium (II)-dichloromethane mixture (0.12 g, 0.14 mmol), potassium carbonate (0.39 g, 2.82 mmol) and DMF (5 mL) to obtain the title compound (0.17 g, 29%) as a white solid.
¹H-NMR (400 MHz, CDCl₃): δ ppm: 2.24 (3H, s), 2.42 (3H, s), 3.85 (3H, s), 3.93 (3H, s), 5.41 (2H, s), 6.51 (1H, d, J = 8.2 Hz), 7.07 (1H, dd, J = 2.0, 8.6 Hz), 7.13 (1H, d, J = 2.0 Hz), 7.29 (1H, ddd, J = 1.2, 3.1, 8.6 Hz), 7.38 (1H, d, J = 7.8 Hz), 7.39 (1H, t, J = 7.8 Hz), 7.69-7.76 (3H, m).

### (4c) 3-({6-[(5,6-Dimethylpyridin-2-yl)oxy]-1-methyl-1H-benzimidazol-2-yl}methoxy)benzoic acid

The reaction and post-treatment were carried out according to Reference Example (1b) using methyl 3-({6-[(5,6-dimethylpyridin-2-yl)oxy]-1-methyl-1H-benzimidazol-2-yl}methoxy)benzoate produced in Reference Example (4b) (0.17 g, 0.41 mmol), a 2 M sodium hydroxide aqueous solution (5 mL) and 1,4-dioxane (10 mL) to obtain the title compound (0.16 g, 99%) as a white solid.
¹H-NMR (400 MHz, DMSO-d₆) : δ ppm: 2.19 (3H, s), 2.25 (3H, s), 3.82 (3H, s), 5.47 (2H, s), 6.66 (1H, d, J = 7.8 Hz), 6.94 (1H, dd, J = 2.4, 8.6 Hz), 7.37 (1H, d, J = 2.0 Hz), 7.38 (1H, ddd, J = 1.2, 2.7, 8.2 Hz), 7.45 (1H, t, J = 7.4 Hz), 7.54 (1H, d, J = 8.2 Hz), 7.58 (1H, dt, J = 1.6, 6.3 Hz), 7.63-7.65 (2H, m), 13.03 (1H, brs); Anal. Calcd for C₂₃H₂₁N₃O₄·0.33H₂O: C, 67.47; H, 5.33; N, 10.26. Found C, 67.40; H, 5.26; N, 10.27.

### (Reference Example 5)

3-({6-[(5-Chloro-3-fluoropyridin-2-yl)oxy]-1-methyl-1H-benzimidazol-2-yl}methoxy)benzoic acid

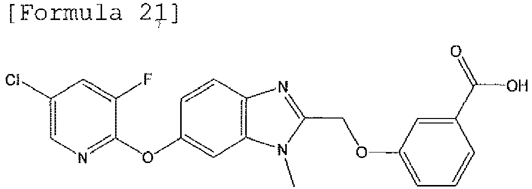

### (5a) Methyl 3-({6-[(5-chloro-3-fluoropyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (15.6 g, 50.0 mmol), 5-chloro-2,3-difluoropyridine (8.22 g, 55.0 mmol), copper iodide (0.95 g, 5.00 mmol), 1,10-phenanthroline (0.90 g, 5.00 mmol), cesium carbonate (48.9 g, 150 mmol) and DMF (200 mL) to obtain the title compound (15.4 g, 70%) as a white solid.
¹H-NMR (500 MHz, CDCl₃): δ ppm: 3.87 (3H, s), 3.93 (3H, s), 5.42 (2H, s), 7.09 (1H, dd, J = 2.4, 8.8 Hz), 7.21 (1H, d, J = 2.0 Hz), 7.30 (1H, ddd, J = 1.0, 2.4, 8.3 Hz), 7.38 (1H, t, J = 8.3 Hz), 7.54 (1H, dd, J = 2.0, 8.8 Hz), 7.70 (1H, dt, J = 1.0, 7.8 Hz), 7.73 (1H, dd, J = 1.5, 2.4 Hz), 7.80 (1H, d, J = 8.8 Hz), 7.88 (1H, d, J = 2.0 Hz).

### (5b) 3-({6-[(5-Chloro-3-fluoropyridin-2-yl)oxy]-1-methyl-1H-benzimidazol-2-yl}methoxy)benzoic acid

The reaction and post-treatment were carried out according to Reference Example (1b) using methyl 3-({6-[(5-chloro-3-fluoropyridin-2-yl)oxy]-1-methyl-1H-benzimidazol-2-yl}methoxy)benzoate produced in Reference Example (5a) (15.4 g, 34.9 mmol), a 2 M sodium hydroxide aqueous solution (100 mL) and THF (200 mL) to obtain the title compound (14.0 g, 94%) as a white solid.
¹H-NMR (400 MHz, DMSO-d₆): δ ppm: 3.83 (3H, s), 5.49 (2H, s), 7.06 (1H, ddd, J = 1.2, 2.4, 8.6 Hz), 7.37-7.40 (1H, m), 7.45 (1H, t, J = 7.4 Hz), 7.52 (1H, d, J = 2.4 Hz), 7.58 (1H, dd, J = 1.6, 7.8 Hz), 7.64 (1H, t, J = 1.2 Hz), 7.68 (1H, d, J = 8.6 Hz), 8.02 (1H, dd, J = 1.2, 2.4 Hz), 8.23 (1H, ddd, J = 1.2, 2.0, 9.8 Hz), 13.06 (1H, s); Anal. Calcd for C₂₁H₁₅ClFN₃O₄: C, 58.96; H, 3.53; N, 9.82. Found C, 58.73; H, 3.40; N, 9.74.

### (Reference Example 6)

3-({6-[(5-Chloro-3-methylpyridin-2-yl)oxy]-1-methyl-1H-benzimidazol-2-yl}methoxy)benzoic acid

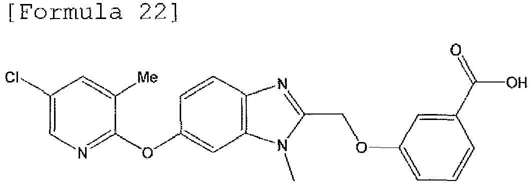

### (6a) Methyl 3-({6-[(5-chloro-3-bromopyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (9.40 g, 30.0 mmol), 3-bromo-5-chloro-2-fluoropyridine (6.90 g, 33.0 mmol), copper iodide (0.57 g, 3.00 mmol), 1,10-phenanthroline (0.54 g, 3.00 mmol), cesium carbonate (29.3 g, 90 mmol) and DMF (90 mL) to obtain the title compound (13.7 g, 91%) as a white solid.
¹H-NMR (400 MHz, CDCl₃): δ ppm: 3.83 (3H, s), 3.89 (3H, s), 5.38 (2H, s), 6.96-7.01 (1H, m), 7.09-7.11 (1H, m), 7.23-7.27 (1H, m), 7.30-7.36 (1H, m), 7.48-7.50 (1H, m), 7.62-7.66 (1H, m), 7.67-7.70 (1H, m), 7.72-7.75 (1H, m), 7.85-7.87 (1H, m).

### (6b) Methyl 3-({6-[(5-chloro-3-methylpyridin-2-yl)oxy]-1-methyl-1H-benzimidazol-2-yl}methoxy)benzoate

The reaction and post-treatment were carried out according to Reference Example (2b) using methyl 3-({6-[(5-chloro-3-bromopyridin-2-yl)oxy]-1-methyl-1H-benzimidazol-2-yl)methoxy)benzoate produced in Reference Example (6a) (10.1 g, 20.0 mmol), trimethylboroxine (50% solution in THF, 6.2 mL, 44.0 mmol), a [1,1'-bis(diphenylphosphino)-ferrocene]dichloropalladium (II)-dichloromethane mixture (0.82 g, 1.00 mmol), potassium carbonate (8.29 g, 60.0 mmol) and DMF (80 mL) to obtain the title compound (6.60 g, 75%) as a white solid.
¹H-NMR (400 MHz, CDCl₃) : δ ppm: 2.41 (3H, s), 3.82 (3H, s), 3.89 (3H, s), 5.38 (2H, s), 6.98-7.00 (1H, m), 7.09-7.11 (1H, m), 7.22-7.27 (1H, m), 7.31-7.37 (1H, m), 7.49-7.50 (1H, m), 7.64-7.66 (1H, m), 7.64-7.69 (1H, m), 7.74 (1H, d, J = 8.6 Hz), 7.84-7.87 (1H, m).

### (6c) 3-({6-[(5-Chloro-3-methylpyridin-2-yl)oxy]-1-methyl-1H-benzimidazol-2-yl}methoxy)benzoic acid

The reaction and post-treatment were carried out according to Reference Example (1b) using methyl 3-({6-[(5-chloro-3-methylpyridin-2-yl)oxy]-1-methyl-1H-benzimidazol-2-yl}methoxy)benzoate produced in Reference Example (6b) (465 mg, 1.06 mmol), a 1 M sodium hydroxide aqueous solution (2.1 mL), THF (10 mL) and methanol (10 mL) to obtain the title compound (230 mg, 51%) as a white solid.
¹H-NMR (400 MHz, DMSO-d₆): δ ppm: 2.32 (3H, s), 3.79 (3H, s), 5.45 (2H, s), 6.94 (1H, dd, J = 2.2. 8.8 Hz), 7.37-7.41 (3H, m), 7.53-7.55 (1H, m), 7.60-7.62 (2H, m), 7.83-7.85 (1H, m), 7.89-7.92 (1H, m), 13.01 (1H, s); Anal. Calcd for C₂₂H₁₈ClN₃O₄: C, 58.96; H, 3.53; N, 9.82. Found C, 58.73; H, 3.40; N, 9.74;
FAB-MS m/z: 424 (M+H)⁺.

### (Reference Example 7)

3-({6-[(3,5-Dichloropyridin-2-yl)oxy]-1-methyl-1H-benzimidazol-2-yl}methoxy)benzoic acid

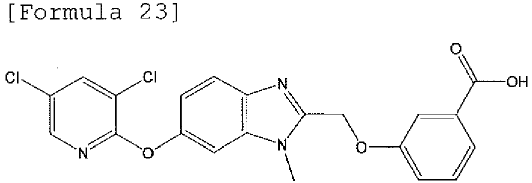

### (7a) Methyl 3-({6-[(3,5-dichloropyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (1e) (7.81 g, 25.0 mmol), 3,5-dichloro-2-fluoropyridine (4.57 g, 27.5 mmol), copper iodide (0.48 g, 2.50 mmol), 1,10-phenanthroline (0.45 g, 2.50 mmol), cesium carbonate (24.44 g, 75.0 mmol) and DMF (100 mL) to obtain the title compound (5.90 g, 53%) as a white solid.
¹H-NMR (400 MHz, CDCl₃): δ ppm: 3.87 (3H, s), 3.93 (3H, s), 5.42 (2H, s), 7.08 (1H, dd, J = 2.0, 8.6 Hz), 7.20 (1H, s), 7.29-7.31 (1H, m), 7.38 (1H, t, J = 8.2 Hz), 7.70 (1H, d, J = 7.4 Hz), 7.73 (1H, s), 7.80 (1H, d, J = 2.4 Hz), 7.82 (1H, brs), 7.96 (1H, d, J = 2.4 Hz).

### (7b) 3-({6-[(3,5-Dichloropyridin-2-yl)oxy]-1-methyl-1H-benzimidazol-2-yl}methoxy)benzoic acid

The reaction and post-treatment were carried out according to Reference Example (1b) using methyl 3-({6-[(3,5-dichloropyridin-2-yl)oxy]-1-methyl-1H-benzimidazol-2-yl}methoxy)benzoate produced in Reference Example (7a) (5.90 g, 12.9 mmol), a 2 M sodium hydroxide aqueous solution (50 mL) and 1,4-dioxane (100 mL) to obtain the title compound (5.30 g, 93%) as a white solid. ¹H-NMR (400 MHz, DMSO-d₆) : δ ppm: 3.83 (3H, s), 5.49 (2H, s), 7.04 (1H, dd, J = 2.4, 9.0 Hz), 7.39 (1H, dd, J = 2.4, 8.2 Hz), 7.45 (1H, t, J = 7.8 Hz), 7.51 (1H, d, J = 2.4 Hz), 7.57 (1H, d, J = 7.4 Hz), 7.64 (1H, s), 7.68 (1H, d, J = 9.0 Hz), 8.12 (1H, dd, J = 0.8, 2.4 Hz), 8.36 (1H, d, J = 2.4 Hz), 13.04 (1H, brs);
Anal. Calcd for C₂₁H₁₅Cl₂N₃O₄·0.25H₂O: C, 56.20; H, 3.48; N, 9.36. Found C, 56.20; H, 3.30; N, 9.53.

### (Reference Example 8)

3-({6-[(5-Fluoro-3-methylpyridin-2-yl)oxy]-1-methyl-1H-benzimidazol-2-yl}methoxy)benzoic acid

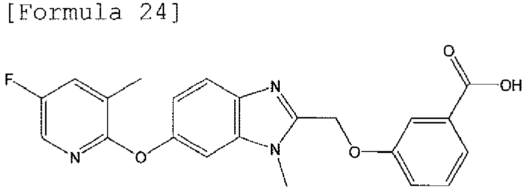

### (8a) 3-Bromo-2,5-difluoropyridine

Sodium nitrite (1.97 g, 28.6 mmol) was added in small portions to a solution of 3-bromo-5-fluoropyridin-2-amine (WO200625783 A1, 3.64 g, 19.1 mmol) in hydrogen fluoride-pyridine (10 mL) at -10°C. After stirring at room temperature for two hours, water (100 mL) and sodium bicarbonate were added to the reaction mixture at 0°C, followed by extraction with ethyl acetate (100 mL). Then, the organic layer was washed with water (100 mL) twice and dried over anhydrous sodium sulfate. After concentration under reduced pressure, the residue was purified by silica gel chromatography (methylene chloride) to obtain the title compound (1.56 g, 42%) as a brown liquid.
¹H-NMR (400 MHz, CDCl₃): δ ppm: 7.78 (1H, dt, J = 2.7, 6.7 Hz), 8.02 (1H, dd, J = 1.6, 2.4 Hz).

### (8b) Methyl 3-({6-[(3-bromo-5-fluoropyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (2.28 g, 7.30 mmol), 3-bromo-2,5-difluoropyridine produced in Reference Example (8a) (1.56 g, 8.03 mmol), copper iodide (0.14 g, 0.73 mmol), 1,10-phenanthroline (0.13 g, 0.73 mmol), cesium carbonate (7.14 g, 21.9 mmol) and DMF (40 mL) to obtain the title compound (1.92 g, 54%) as a white solid.
¹H-NMR (500 MHz, DMSO-d₆): δ ppm: 3.83 (3H, s), 3.85 (3H, s), 5.50 (2H, s), 7.00 (1H, dd, J = 2.4, 8.6 Hz), 7.43 (1H, ddd, J = 1.2, 2.4, 8.2 Hz), 7.45-7.50 (2H, m), 7.59 (1H, dt, J = 1.2, 7.8 Hz), 7.65-7.67 (2H, m), 8.13 (1H, d, J = 2.7 Hz), 8.38 (1H, dd, J = 2.7, 7.4 Hz).

### (8c) Methyl 3-({6-[(5-fluoro-3-methylpyridin-2-yl)oxy]-1-methyl-1H-benzimidazol-2-yl}methoxy)benzoate

The reaction and post-treatment were carried out according to Reference Example (2b) using methyl 3-({6-[(3-bromo-5-fluoropyridin-2-yl)oxy]-1-methyl-1H-benzimidazol-2-yl}methoxy)benzoate produced in Reference Example (8b) (1.92 g, 3.95 mmol), trimethylboroxine (50% solution in THF, 2.23 mL, 7.90 mmol), a [1,1'-bis(diphenylphosphino)-ferrocene]dichloropalladium (II)-dichloromethane mixture (0.32 g, 0.39 mmol), potassium carbonate (1.09 g, 7.90 mmol) and DMF (40 mL) to obtain the title compound (0.99 g, 60%) as a white solid.
¹H-NMR (500 MHz, CDCl₃) δ ppm: 2.41 (3H, s), 3.85 (3H, s), 3. 93 (3H, s), 5.41 (2H, s), 7.03 (1H, dd, J = 1. 5, 8.8 Hz), 7.13 (1H, d, J = 2.0 Hz), 7.30 (1H, dd, J = 2.9, 8.3 Hz), 7.34 (1H, dd, J = 1.5, 7.3 Hz), 7.37 (1H, t, J = 8.3 Hz), 7.69 (1H, d, J = 7.3 Hz), 7.73 (1H, s), 7.77 (1H, d, J = 8.8 Hz), 7.81 (1H, d, J = 2.9 Hz).

### (8d) 3-({6-[(5-Fluoro-3-methylpyridin-2-yl)oxy]-1-methyl-1H-benzimidazol-2-yl}methoxy)benzoic acid

The reaction and post-treatment were carried out according to Reference Example (1b) using methyl 3-({6-[(5-fluoro-3-methylpyridin-2-yl)oxy]-1-methyl-1H-benzimidazol-2-yl}methoxy)benzoate produced in Reference Example (8c) (0.99 g, 2.35 mmol), a 1 M sodium hydroxide aqueous solution (10 mL), 1,4-dioxane (10 mL) and methanol (10 mL) to obtain the title compound (0.91 g, 95%) as a white solid.
¹H-NMR (400 MHz, DMSO-d₆): δ ppm: 2.35 (3H, s), 3.81 (3H, s), 5.47 (2H, s), 6.95 (1H, dd, J = 2.4, 8.6 Hz), 7.36-7.38 (2H, m), 7.44 (1H, t, J = 7.4 Hz), 7.57 (1H, d, J = 7.4 Hz), 7.62-7.64 (2H, m), 7.75 (1H, dd, J = 2.7, 8.6 Hz), 7.89 (1H, dd, J = 0.8, 2.7 Hz), 13.04 (1H, brs); Anal. Calcd for C₂₂H₁₈FN₃O₄·0.5H₂O: C, 63.46; H, 4.60; N, 10.09. Found C, 63.74; H, 4.26; N, 10.26.

### (Reference Example 9)

3-({6-[(3-Fluoro-5-methylpyridin-2-yl)oxy]-1-methyl-1H-benzimidazol-2-yl}methoxy)benzoic acid

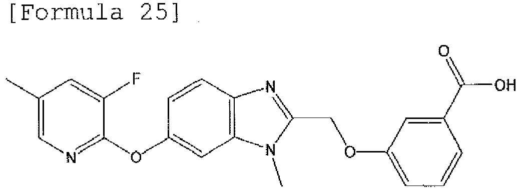

### (9a) 5-Bromo-2,3-difluoropyridine

The reaction and post-treatment were carried out according to Reference Example (8a) using 5-bromo-3-fluoropyridin-2-amine (WO20078478 A1) (8.42 g, 44.1 mmol), sodium nitrite (4.56 g, 66.1 mmol) and hydrogen fluoride-pyridine (15 mL) to obtain the title compound (8.55 g, 91%) as a colorless liquid.
¹H-NMR (500 MHz, CDCl₃): δ ppm: 7.74 (1H, dt, J = 2.0, 8.3 Hz), 8.08 (1H, t, J = 2.0 Hz).

### (9b) Methyl 3-({6-[(5-bromo-3-fluoropyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (4.06 g, 13.0 mmol), 5-Bromo-2,3-difluoropyridine produced in Reference Example (9a) (2.77 g, 14.3 mmol), copper iodide (0.25 g, 1.30 mmol), 1,10-phenanthroline (0.23 g, 1.30 mmol), cesium carbonate (12.71 g, 39.0 mmol) and DMF (65 mL) to obtain the title compound (4.14 g, 66%) as a white solid.
¹H-NMR (500 MHz, CDCl₃): δ ppm: 3.87 (3H, s), 3.93 (3H, s), 5.41 (2H, brs), 7.10 (1H, brs), 7.23 (1H, brs), 7.31 (1H, d, J = 8.3 Hz), 7.38 (1H, t, J = 7.8 Hz), 7.67 (1H, dd, J = 2.0, 8.8 Hz), 7.70 (1H, d, J = 7.8 Hz), 7.73 (1H, s), 7.80 (1H, brs), 7.96 (1H, d, J = 2.0 Hz).

### (9c) Methyl 3-({6-[(3-fluoro-5-methylpyridin-2-yl)oxy]-1-methyl-1H-benzimidazol-2-yl}methoxy)benzoate

The reaction and post-treatment were carried out according to Reference Example (2b) using methyl 3-({6-[(5-bromo-3-fluoropyridin-2-yl)oxy]-1-methyl-1H-benzimidazol-2-yl}methoxy)benzoate produced in Reference Example (9b) (4.14 g, 8.51 mmol), trimethylboroxine (50% solution in THF, 4.80 mL, 17.0 mmol), a [1,1'-bis(diphenylphosphino)-ferrocene]dichloropalladium (II)-dichloromethane mixture (0.70 g, 0.85 mmol), potassium carbonate (2.35 g, 17.0 mmol) and DMF (80 mL) to obtain the title compound (2.02 g, 56%) as a white solid.
¹H-NMR (500 MHz, CDCl₃): δ ppm: 2.31 (3H, s), 3.86 (3H, s), 3.93 (3H, s), 5.42 (2H, s), 7.09 (1H, dd, J = 2.0, 8.8 Hz), 7.19 (1H, d, J = 2.0 Hz), 7.30 (1H, ddd, J = 1.0, 2.9, 8.3 Hz), 7.32-7.35 (1H, m), 7.38 (1H, t, J = 7.8 Hz), 7.69 (1H, dt, J = 1.5, 7.3 Hz), 7.72-7.73 (2H, m), 7.78 (1H, d, J = 8.8 Hz).

### (9d) 3-({6-[(3-Fluoro-5-methylpyridin-2-yl)oxy]-1-methyl-1H-benzimidazol-2-yl}methoxy)benzoic acid

The reaction and post-treatment were carried out according to Reference Example (1b) using methyl 3-({6-[(3-fluoro-5-methylpyridin-2-yl)oxy]-1-methyl-1H-benzimidazol-2-yl}methoxy)benzoate produced in Reference Example (9c) (2.02 g, 4.79 mmol), a 1 M sodium hydroxide aqueous solution (25 mL) and methanol (50 mL) to obtain the title compound (1.98 g, 98%) as a white solid. ¹H-NMR (500 MHz, DMSO-d₆) δ ppm: 2.27 (3H, s), 3.82 (3H, s), 5.47 (2H, s), 7.00 (1H, dd, J = 2.4, 8.8 Hz), 7.38 (1H, dd, J = 1.5, 7.8 Hz), 7.42 (1H, d, J = 2.4 Hz), 7.44 (1H, t, J = 8.3 Hz), 7.56 (1H, dt, J = 1.5, 7.3 Hz), 7.63-7.65 (2H, m), 7.73 (1H, dd, J = 1.5, 11.2 Hz), 7.75 (1H, s), 13.01 (1H, brs).

### (Reference Example 10)

3-({6-[(3,5-Dimethylpyridin-2-yl)oxy]-1-methyl-1H-benzimidazol-2-yl}methoxy)benzoic acid

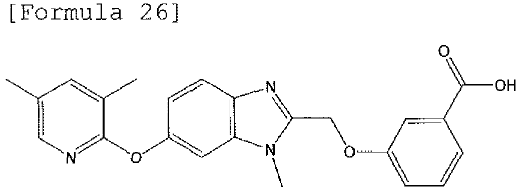

### (10a) Methyl 3-({6-[(3,5-dibromopyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (2.50 g, 8.00 mmol), 3,5-dibromo-2-fluoropyridine (2.24 g, 8.81 mmol), copper iodide (0.15 g, 0.80 mmol), 1,10-phenanthroline (0.14 g, 0.80 mmol), cesium carbonate (7.82 g, 24.0 mmol) and DMF (40 mL) to obtain the title compound (3.24 g, 74%) as a white solid.
¹H-NMR (400 MHz, DMSO-d₆): δ ppm: 3.83 (3H, s), 3.85 (3H, s), 5.50 (2H, s), 7.02 (1H, dd, J = 2.4, 8.6 Hz), 7.43 (1H, ddd, J = 1.2, 2.7, 8.2 Hz), 7.46-7.50 (2H, m), 7.59 (1H, dt, J = 1.6, 7.4 Hz), 7.66-7.68 (2H, m), 8.20 (1H, d, J = 2.0 Hz), 8.52 (1H, d, J = 2.4 Hz).

### (10b) Methyl 3-({6-[(3,5-dimethylpyridin-2-yl)oxy]-1-methyl-1H-benzimidazol-2-yl}methoxy)benzoate

The reaction and post-treatment were carried out according to Reference Example (2b) using methyl 3-({6-[(3,5-dibromopyridin-2-yl)oxy]-1-methyl-1H-benzimidazol-2-yl}methoxy)benzoate produced in Reference Example (10a) (5.47 g, 10.0 mmol), trimethylboroxine (50% solution in THF, 11.28 mL, 40.0 mmol), a [1,1'-bis(diphenylphosphino)-ferrocene]dichloropalladium (II)-dichloromethane mixture (0.82 g, 1.00 mmol), potassium carbonate (5.53 g, 40.0 mmol) and DMF (100 mL) to obtain the title compound (3.80 g, 91%) as a white solid.
¹H-NMR (400 MHz, CDCl₃): δ ppm: 2.25 (3H, s), 2.36 (3H, s), 3.84 (3H, s), 3.93 (3H, s), 5.41 (2H, s), 7.02 (1H, dd, J = 2.0, 8.6 Hz), 7.12 (1H, d, J = 2.0 Hz), 7.28-7.31 (1H, m), 7.35-7.39 (2H, m), 7.69 (1H, dt, J = 1.2, 7.4 Hz), 7.72-7.79 (3H, m).

### (10c) 3-({6-[(3,5-Dimethylpyridin-2-yl)oxy]-1-methyl-1H-benzimidazol-2-yl}methoxy)benzoic acid

The reaction and post-treatment were carried out according to Reference Example (1b) using methyl 3-({6-[(3,5-dimethylpyridin-2-yl)oxy]-1-methyl-1H-benzimidazol-2-yl}methoxy)benzoate produced in Reference Example (10b) (0.96 g, 2.30 mmol), a 1 M sodium hydroxide aqueous solution (50 mL) and methanol (50 mL) to obtain the title compound (0.85 g, 92%) as a white solid.
¹H-NMR (400 MHz, DMSO-d₆) : δ ppm: 2.20 (3H, s), 2.30 (3H, s), 3.81 (3H, s), 5.47 (2H, s), 6.92 (1H, dd, J = 2.4, 8.6 Hz), 7.32 (1H, d, J = 2.4 Hz), 7.36 (1H, dd, J = 1.6, 7.4 Hz), 7.44 (1H, t, J = 7.4 Hz), 7.54-7.63 (4H, m), 7.72 (1H, s), 13.04 (1H, brs);
Anal. Calcd for C₂₃H₂₁N₃O₄: C, 68.47; H, 5.25; N, 10.42. Found C, 68.29; H, 5.17; N, 10.41.

### (Reference Example 11)

3-({6-[(5-Ethyl-3-methylpyridin-2-yl)oxy]-1-methyl-1H-benzimidazol-2-yl}methoxy)benzoic acid

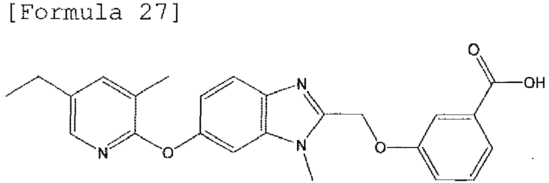

### (11a) Methyl 3-({6-[(5-bromo-3-methylpyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (3.12 g, 10.0 mmol), 5-bromo-2-fluoro-3-methylpyridine (2.09 g, 11.0 mmol), copper iodide (0.19 g, 1.00 mmol), 1,10-phenanthroline (0.18 g, 1.00 mmol), cesium carbonate (9.77 g, 30.0 mmol) and DMF (50 mL) to obtain the title compound (0.65 g, 14%) as a white solid.
¹H-NMR (500 MHz, CDCl₃): δ ppm: 2.39 (3H, s), 3.86 (3H, s), 3.93 (3H, s), 5.42 (2H, s), 7.03 (1H, dd, J = 2.4, 8.8 Hz), 7.15 (1H, d, J = 2.0 Hz), 7.31 (1H, dd, J = 2.0, 7.3 Hz), 7.38 (1H, t, J = 7.8 Hz), 7.66-7.67 (1H, m), 7.70 (1H, d, J = 7.8 Hz), 7.73 (1H, t, J = 2.0 Hz), 7.78 (1H, d, J = 8.8 Hz), 8.00 (1H, d, J = 2.0 Hz).

### (11b) Methyl 3-({6-[(5-ethyl-3-methylpyridin-2-yl)oxy]-1-methyl-1H-benzimidazol-2-yl}methoxy)benzoate

The reaction and post-treatment were carried out according to Reference Example (2b) using methyl 3-({6-[(5-bromo-3-methylpyridin-2-yl)oxy]-1-methyl-1H-benzimidazol-2-yl}methoxy)benzoate produced in Reference Example (11a) (0.65 g, 1.35 mmol), triethylborane (1.0 M solution in THF, 2.70 mL, 2.70 mmol), a [1,1'-bis(diphenylphosphino)-ferrocene]dichloropalladium (II)-dichloromethane mixture (0.11 g, 0.13 mmol), potassium carbonate (0.37 g, 2.70 mmol) and DMF (10 mL) to obtain the title compound (0.58 g, 99%) as a pale yellow oil. ¹H-NMR (400 MHz, CDCl₃): δ ppm: 1.23 (3H, t, J = 8.2 Hz), 2.37 (3H, s), 2.58 (2H, q, J = 7.8 Hz), 3.85 (3H, s), 3.93 (3H, s), 5.42 (2H, s), 7.04 (1H, dd, J = 2.4, 8.6 Hz), 7.14 (1H, d, J = 2.0 Hz), 7.29-7.32 (1H, m), 7.37 (1H, d, J = 7.8 Hz), 7.40 (1H, d, J = 2.7 Hz), 7.69 (1H, d, J = 7.4 Hz), 7.72 (1H, t, J = 2.0 Hz), 7.76 (1H, d, J = 8.6 Hz), 7.81 (1H, d, J = 2.4 Hz).

### (11c) 3-({6-[(5-Ethyl-3-methylpyridin-2-yl)oxy]-1-methyl-1H-benzimidazol-2-yl}methoxy)benzoic acid

The reaction and post-treatment were carried out according to Reference Example (1b) using methyl 3-({6-[(5-ethyl-3-methylpyridin-2-yl)oxy]-1-methyl-1H-benzimidazol-2-yl}methoxy)benzoate produced in Reference Example (11b) (0.58 g, 1.34 mmol), a 1 M sodium hydroxide aqueous solution (5 mL) and methanol (10 mL) to obtain the title compound (0.43 g, 77%) as a white solid. ¹H-NMR (400 MHz, DMSO-d₆): δ ppm: 1.16 (3H, t, J = 7.8 Hz), 2.31 (3H, s), 2.52 (2H, q, J = 7.8 Hz), 3.81 (3H, s), 5.47 (2H, s), 6.93 (1H, dd, J = 2.0, 8.6 Hz), 7.34 (1H, d, J = 2.4 Hz), 7.39 (1H, ddd, J = 0.8, 2.4, 8.2 Hz), 7.45 (1H, t, J = 7.4 Hz), 7.56-7.58 (2H, m), 7.61 (1H, d, J = 9.0 Hz), 7.64 (1H, dd, J = 1.6, 2.7 Hz), 7.73 (1H, d, J = 2.4 Hz), 12.98 (1H, s).

### (Reference Example 12)

3-({6-[(3-Ethyl-5-methylpyridin-2-yl)oxy]-1-methyl-1H-benzimidazol-2-yl}methoxy)benzoic acid

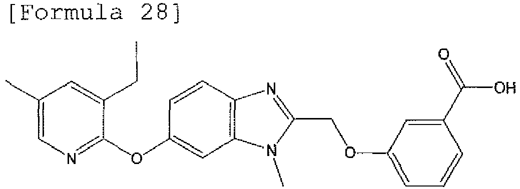

### (12a) Methyl 3-({6-[(3-bromo-5-methylpyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (3.12 g, 10.0 mmol), 3-bromo-2-fluoro-5-methylpyridine (2.09 g, 11.0 mmol), copper iodide (0.19 g, 1.00 mmol), 1,10-phenanthroline (0.18 g, 1.00 mmol), cesium carbonate (9.77 g, 30.0 mmol) and DMF (50 mL) to obtain the title compound (0.65 g, 14%) as a white solid.
¹H-NMR (500 MHz, CDCl₃) : δ ppm: 2.28 (3H, s), 3.86 (3H, s), 3.93 (3H, s), 5.42 (2H, s), 7.08 (1H, dd, J = 2.4, 8.8 Hz), 7.19 (1H, d, J = 2.0 Hz), 7.31 (1H, dd, J = 2.9, 8.3 Hz), 7.38 (1H, t, J = 8.3 Hz), 7.70 (1H, d, J = 7.8 Hz), 7.73 (1H, s), 7.77-7.80 (2H, m), 7.87 (1H, s).

### (12b) Methyl 3-({6-[(3-ethyl-5-methylpyridin-2-yl)oxy]-1-methyl-1H-benzimidazol-2-yl}methoxy)benzoate

The reaction and post-treatment were carried out according to Reference Example (2b) using methyl 3-({6-[(3-bromo-5-methylpyridin-2-yl)oxy]-1-methyl-1H-benzimidazol-2-yl}methoxy)benzoate produced in Reference Example (12a) (0.65 g, 1.35 mmol), triethylborane (1.0 M solution in THF, 2.70 mL, 2.70 mmol), a [1,1'-bis(diphenylphosphino)-ferrocene]dichloropalladium (II)-dichloromethane mixture (0.11 g, 0.13 mmol), potassium carbonate (0.37 g, 2.70 mmol) and DMF (10 mL) to obtain the title compound (0.23 g, 40%) as a white solid.
¹H-NMR (500 MHz, CDCl₃): δ ppm: 1.31 (3H, t, J = 6.4 Hz), 2.27 (3H, s), 2.76 (2H, q, J = 7.3 Hz), 3.84 (3H, s), 3.93 (3H, s), 5.42 (2H, s), 7.03 (1H, dd, J = 2.4, 7.8 Hz), 7.12 (1H, s), 7.29-7.31 (1H, m), 7.37 (1H, d, J = 7.8 Hz), 7.39 (1H, s), 7.69 (1H, d, J = 7.8 Hz), 7.72 (1H, t, J = 1.5 Hz), 7.76 (1H, d, J = 8.8 Hz), 7.80 (1H, s).

### (12c) 3-({6-[(3-Ethyl-5-methylpyridin-2-yl)oxy]-1-methyl-1H-benzimidazol-2-yl}methoxy)benzoic acid

The reaction and post-treatment were carried out according to Reference Example (1b) using methyl 3-({6-[(3-ethyl-5-methylpyridin-2-yl)oxy]-1-methyl-1H-benzimidazol-2-yl}methoxy)benzoate produced in Reference Example (12b) (0.23 g, 0.53 mmol), a 1 M sodium hydroxide aqueous solution (5 mL) and methanol (10 mL) to obtain the title compound (0.15 g, 68%) as a white solid.
¹H-NMR (500 MHz, DMSO-d₆) : δ ppm: 1.25 (3H, t, J = 7.8 Hz), 2.22 (3H, s), 2.69 (2H, q, J = 7.3 Hz), 3.82 (3H, s), 5.48 (2H, s), 6.92 (1H, dd, J = 2.4, 8.8 Hz), 7.33 (1H, d, J = 2.0 Hz), 7.39 (1H, dd, J = 2.4, 8.3 Hz), 7.45 (1H, t, J = 7.8 Hz), 7.55 (1H, d, J = 2.4 Hz), 7.58 (1H, d, J = 7.3 Hz), 7.62 (1H, d, J = 8. 3 Hz), 7.64 (1H, dd, J = 1. 5, 2.9 Hz), 7.73 (1H, d, J = 2.0 Hz), 13.02 (1H, s).

### (Reference Example 13)

3-({6-[(3,6-Difluoropyridin-2-yl)oxy]-1-methyl-1H-benzimidazol-2-yl}methoxy)benzoic acid

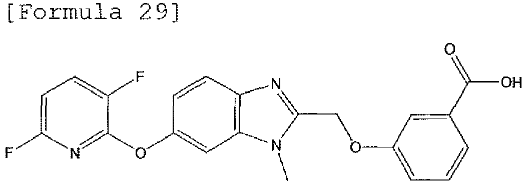

### (13a) Methyl 3-({6-[(3,6-difluoropyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (3.12 g, 10.0 mmol), 2,3,6-trifluoropyridine (1.46 g, 11.0 mmol), copper iodide (0.19 g, 1.00 mmol), 1,10-phenanthroline (0.18 g, 1.00 mmol), cesium carbonate (9.77 g, 30.0 mmol) and DMF (50 mL) to obtain the title compound (3.02 g, 71%) as a white solid.
¹H-NMR (400 MHz, CDCl₃): δ ppm: 3.88 (3H, s), 3.93 (3H, s), 5.42 (2H, s), 6.58 (1H, ddd, J = 2.4, 3.5, 8.6 Hz), 7.11 (1H, dd, J = 2.0, 8.6 Hz), 7.22 (1H, d, J = 2.4 Hz), 7.31 (1H, ddd, J = 0.8, 2.7, 8.2 Hz), 7.39 (1H, t, J = 7.8 Hz), 7.59 (1H, dt, J = 5.9, 8.2 Hz), 7.70 (1H, dt, J = 1.2, 7.8 Hz), 7.73 (1H, dd, J = 1.6, 2.4 Hz), 7.79 (1H, d, J = 8.6 Hz).

### (13b) 3-({6-[(3,6-Difluoropyridin-2-yl)oxy]-1-methyl-1H-benzimidazol-2-yl}methoxy)benzoic acid

The reaction and post-treatment were carried out according to Reference Example (1b) using methyl 3-({6-[(3,6-difluoropy.idin-2-yl)oxy]-1-methyl-1H-benzimidazol-2-yl}methoxy)benzoate produced in Reference Example (13a) (3.02 g, 7.10 mmol), a 2 M sodium hydroxide aqueous solution (10 mL) and 1,4-dioxane (20 mL) to obtain the title compound (2.52 g, 86%) as a white solid.
¹H-NMR (400 MHz, DMSO-d₆): δ ppm: 3.84 (3H, s), 5.48 (2H, s), 6.93 (1H, dt, J = 2.0, 8.6 Hz), 7.08 (1H, dd, J = 2.0, 8.6 Hz), 7.38 (1H, dd, J = 1.6, 8.2 Hz), 7.45 (1H, t, J = 7.4 Hz), 7.55 (1H, d, J = 2.4 Hz), 7.57 (1H, d, J = 7.8 Hz), 7.64 (1H, s), 7.69 (1H, d, J = 8.6 Hz), 8.09 (1H, dt, J = 6.3, 8.6 Hz), 13.08 (1H, brs).

### (Reference Example 14)

3-({6-[(4-Methoxy-3,5-dimethylpyridin-2-yl)oxy]-1-methyl-1H-benzimidazol-2-yl}methoxy)benzoic acid

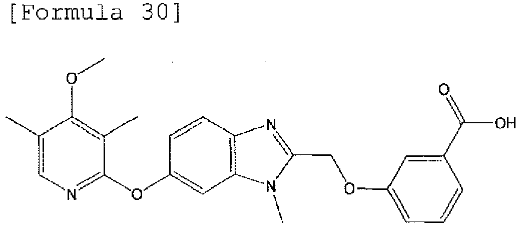

### (14a) 3,5-Dibromo-4-chloropyridin-2-amine

A solution of 4-chloropyridin-2-amine (8.16 g, 63.5 mmol) and N-bromosuccinimide (23.7 g, 133 mmol) in dichloromethane (200 mL) was stirred at room temperature for one hour. The insoluble matter was separated by filtration and the filtrate was concentrated under reduced pressure. Then, the residue was purified by silica gel chromatography (methylene chloride/ethyl acetate, 1:1) to obtain the title compound (18.2 g, 56%) as a white solid.
¹H-NMR (400 MHz, CDCl₃): δ ppm: 5.08 (2H, brs), 8.13 (1H, s).

### (14b) 3,5-Dibromo-4-chloro-2-fluoropyridine

The reaction and post-treatment were carried out according to Reference Example (8a) using 3,5-dibromo-4-chloropyridin-2-amine produced in Reference Example (14a) (10.1 g, 35.4 mmol), sodium nitrite (3.66 g, 53.1 mmol) and hydrogen fluoride-pyridine (50 mL) to obtain the title compound (8.50 g, 83%) as a pale yellow oil.
¹H-NMR (400 MHz, CDCl₃) : δ ppm: 8.32 (1H, s).

### (14c) Methyl 3-({6-[(3,5-dibromo-4-chloropyridin-2-yl)oxy]-1-methyl-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (1.69 g, 5.00 mmol), 3,5-dibromo-4-chloro-2-fluoropyridine produced in Reference Example (14b) (1.59 g, 5.50 mmol), copper iodide (0.10 g, 0.50 mmol), 1,10-phenanthroline (0.09 g, 0.50 mmol), cesium carbonate (4.89 g, 15.0 mmol) and DMF (30 mL) to obtain the title compound (1.56 g, 54%) as a white solid.
¹H-NMR (500 MHz, CDCl₃) : δ ppm: 3.87 (3H, s), 3.93 (3H, s), 5.42 (2H, s), 7.05-7.07 (1H, m), 7.18 (1H, d, J = 2.0 Hz), 7.28-7.31 (1H, m), 7.38 (1H, t, J = 7.8 Hz), 7.70 (1H, d, J = 7.8 Hz), 7.72-7.74 (1H, m), 7.81 (1H, d, J = 8.8 Hz), 8.16 (1H, s).

### (14d) Methyl 3-({6-[(3,5-dibromo-4-methoxypyridin-2-yl)oxy]-1-methyl-1H-benzimidazol-2-yl}methoxy)benzoate

The reaction and post-treatment were carried out according to Reference Example (3b) using methyl 3-({6-[(3,5-dibromo-4-chloropyridin-2-yl)oxy]-1-methyl-1H-benzimidazol-2-yl}methoxy)benzoate produced in Reference Example (14c) (1.56 g, 2.68 mmol), sodium methoxide (5.0 M solution in methanol, 5.36 mL, 26.8 mmol), water (5 mL) and methanol (100 mL) to obtain the title compound (0.13 g, 8%) as a white solid.
¹H-NMR (400 MHz, CDCl₃) : δ ppm: 3.87 (3H, s), 3.93 (3H, s), 4.05 (3H, s), 5.42 (2H, s), 7.07 (1H, dd, J = 2.0, 8.6 Hz), 7.18 (1H, d, J = 2.4 Hz), 7.28-7.31 (1H, m), 7.38 (1H, t, J = 7.8 Hz), 7.69 (1H, d, J = 7.8 Hz), 7.72-7.73 (1H, m), 7.80 (1H, d, J = 8.6 Hz), 8.09 (1H, s).

### (14e) Methyl 3-({5-[(4-methoxy-3,5-dimethylpyridin-2-yl)oxy]-1-methyl-1H-benzimidazol-2-yl}methoxy)benzoate

The reaction and post-treatment were carried out according to Reference Example (2b) using methyl 3-({6-[(3,5-dibromo-4-methoxypyridin-2-yl)oxy]-1-methyl-1H-benzimidazol-2-yl}methoxy)benzoate produced in Reference Example (14d) (0.13 g, 0.23 mmol), trimethyl boroxine (50% solution in THF, 0.25 mL, 0.90 mmol), a [1,1'-bis(diphenylphosphino)-ferrocene]dichloropalladium (II)-dichloromethane mixture (0.02 g, 0.02 mmol), potassium carbonate (0.12 g, 0.90 mmol) and DMF (10 mL) to obtain the title compound (57 mg, 57%) as a white solid.
¹H-NMR (500 MHz, CDCl₃) : δ ppm: 2.20 (3H, s), 2.31 (3H, s), 3.84 (3H, s), 3.84 (3H, s), 3.93 (3H, s), 5.42 (2H, s), 7.03 (1H, dd, J = 2.0, 8.8 Hz), 7.13 (1H, d, J = 2.4 Hz), 7.28-7.31 (1H, m), 7.37 (1H, t, J = 7.8 Hz), 7.69 (1H, dt, J = 1.0, 7.3 Hz), 7.72 (1H, dd, J = 1.5, 2.4 Hz), 7.76 (1H, d, J = 8.8 Hz), 7.78 (1H, s).

### (14f) 3-({6-[(4-Methoxy-3,5-dimethylpyridin-2-yl)oxy]-1-methyl-1H-benzimidazol-2-yl}methoxy)benzoic acid

The reaction and post-treatment were carried out according to Reference Example (1b) using methyl 3-({6-[(4-methoxy-3,5-dimethylpyridin-2-yl)oxy]-1-methyl-1H-benzimidazol-2-y1}methoxy)benzoate produced in Reference Example (14e) (57 mg, 0.13 mmol), a 1 M sodium hydroxide aqueous solution (2 mL) and methanol (2 mL) to obtain the title compound (37 mg, 67%) as a white solid.
¹H-NMR (400 MHz, DMSO-d₆): δ ppm: 2.14 (3H, s), 2.23 (3H, s), 3.79 (3H, s), 3.81 (3H, s), 5.47 (2H, s), 6.92 (1H, dd, J = 0.8, 8.6 Hz), 7.33 (1H, d, J = 2.4 Hz), 7.38 (1H, d, J = 8.2 Hz), 7.44 (1H, t, J = 7.4 Hz) , 7.57 (1H, d, J = 7.8 Hz) , 7. 61 (1H, d, J = 8.6 Hz), 7.63 (1H, s), 7. 73 (1H, d, J = 0.8 Hz), 13.05 (1H, brs).

### (Reference Example 15)

3-({1-Methyl-6-[(3,5,6-trimethylpyridin-2-yl)oxy]-1H-benzimidazol-2-yl}methoxy)benzoic acid

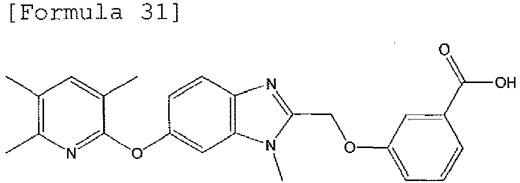

### (15a) 3,5,6-Tribromopyridin-2-amine

The reaction and post-treatment were carried out according to Reference Example (14a) using 6-bromopyridin-2-amine (1.73 g, 10.0 mmol), N-bromosuccinimide (3.74 g, 21.0 mmol) and dichloromethane (50 mL) to obtain the title compound (2.34 g, 71%) as a pale brown solid.
¹H-NMR (400 MHz, CDCl₃) : δ ppm: 5.06 (2H, brs), 7.79 (1H, s).

### (15b) 2,3,5-Tribromo-6-fluoropyridine

The reaction and post-treatment were carried out according to Reference Example (8a) using 3,5,6-tribromopyridin-2-amine produced in Reference Example (15a) (2.34 g, 7.07 mmol), sodium nitrite (0.73 g, 10.6 mmol) and hydrogen fluoride-pyridine (5 mL) to obtain the title compound (1.98 g, 84%) as a pale yellow oil. ¹H-NMR (400 MHz, CDCl₃): δ ppm: 8.15 (1H, d, J = 7.4 Hz).

### (15c) Methyl 3-({1-methyl-6-[(3,5,6-tribromopyridin-2-yl)oxy]-1H-benzimidazol-2-yl}methoxy)benzoate

The reaction and post-treatment were carried out according to Reference Example (1a) using methyl 3-[(6-hydroxy-1-methyl-1H-benzimidazol-2-yl)methoxy]benzoate (1.69 g, 5.40 mmol), 2,3,5-tribromo-6-fluoropyridine produced in Reference Example (15b) (1.98 g, 5.94 mmol), copper iodide (0.10 g, 0.54 mmol), 1,10-phenanthroline (0.10 g, 0.54 mmol), cesium carbonate (5.28 g, 16.2 mmol) and DMF (30 mL) to obtain the title compound (2.92 g, 86%) as a white solid.
¹H-NMR (400 MHz, CDCl₃) : δ ppm: 3.88 (3H, s), 3.93 (3H, s), 5.43 (2H, s), 7.08 (1H, dd, J = 2.4, 9.0 Hz), 7.21 (1H, d, J = 2.0 Hz), 7.29-7.32 (1H, m), 7.39 (1H, t, J = 7.8 Hz), 7.70 (1H, dt, J = 1.6, 6.3 Hz), 7.74 (1H, dd, J = 1.6, 2.4 Hz), 7.79 (1H, d, J = 9.0 Hz), 8.10 (1H, s).

### (15d) Methyl 3-({1-methyl-6-[(3,5,6-trimethylpyridin-2-yl)oxy]-1H-benzimidazol-2-yl}methoxy)benzoate

The reaction and post-treatment were carried out according to Reference Example (2b) using methyl 3-({1-methyl-6-[(3,5,6-tribromopyridin-2-yl)oxy]-1H-benzimidazol-2-yl}methoxy)benzoate produced in Reference Example (15c) (2.92 g, 4.66 mmol), trimethylboroxine (50% solution in THF, 3.89 mL, 14.0 mmol), a [1,1'-bis(diphenylphosphino)-ferrocene]diohloropalladium (II)-dichloromethane mixture (0.38 g, 0.47 mmol), potassium carbonate (1.93 g, 14.0 mmol) and DMF (50 mL) to obtain the title compound (1.30 g, 65%) as a white solid. ¹H-NMR (400 MHz, CDCl₃) : δ ppm: 2.22 (3H, s), 2.25 (3H, s), 2.30 (3H, s), 3.82 (3H, s), 3.93 (3H, s), 5.40 (2H, s), 6.99 (1H, dd, J = 2.4, 9.0 Hz), 7.04 (1H, d, J = 2.4 Hz), 7.29-7.31 (2H, m), 7.38 (1H, t, J = 7.4 Hz), 7.69 (1H, dt, J = 1.2, 7.8 Hz), 7.70 (1H, d, J = 8.6 Hz), 7.73 (1H, dd, J = 1.6, 2.7 Hz).

### (15e) 3-({1-Methyl-6-[(3,5,6-trimethylpyridin-2-yl)oxy]-1H-benzimidazol-2-yl}methoxy)benzoic acid

The reaction and post-treatment were carried out according to Reference Example (1b) using methyl 3-({1-methyl-6-[(3,5,6-trimethylpyridin-2-yl)oxy]-1H-benzimidazol-2-yl}methoxy)benzoate produced in Reference Example (15d) (1.30 g, 3.01 mmol), a 1 M sodium hydroxide aqueous solution (100 mL), 1,4-dioxane (100 mL) and methanol (100 mL) to obtain the title compound (1.13 g, 93%) as a white solid.
¹H-NMR (400 MHz, DMSO-d₆): δ ppm: 2.15 (3H, s), 2.16 (3H, s), 2.23 (3H, s), 3.80 (3H, s), 5.45 (2H, s), 6.88 (1H, dd, J = 2.4, 8.6 Hz), 7.27 (1H, d, J = 2.4 Hz), 7.38 (1H, ddd, J = 1.2, 2.7, 8.2 Hz), 7.74 (1H, s), 7.74 (1H, t, J = 7.8 Hz), 7.57 (1H, d, J = 7.4 Hz), 7.60 (1H, d, J = 9.0 Hz), 7.63 (1H, dd, J = 1.6, 2.4 Hz), 13.03 (1H, brs);
Anal. Calcd for C₂₄H₂₃N₃O₄·0.25H₂O: C, 68.31; H, 5.61; N, 9.96. Found C, 68.58; H, 5.49; N, 9.95.

### (Test Example 1)

### Hypoglycemic effect

Six-week-old male KK mice were purchased from CLEA Japan, Inc. and then were fed until 15 to 20 weeks old to develop diabetes. The mice were individually fed during the adaptation period and the test period, and water and feed (FR2, Funabashi Farm) were freely ingested.

At the start of the experiment, after body weight measurement, blood was collected from the tail vein of the mice into a heparin-coated glass tube and centrifuged, and then plasma was separated. The glucose level in the plasma was measured by Glucoloader GXT (A&T Corp.), and individuals having a plasma glucose level of about 350 mg/dl or more were selected. The mice were grouped, each group having 3 to 4 mice, to make the average body weight and the average plasma glucose level similar. Each compound was administered to a compound group with a diet admixture containing 0.03% of the compound. A separate group in which the mice were fed only with diet was a control group.

The experiment period (drug administration period) was three days. The grouping day was the 0th day. On the 3rd day, the body weight was measured and blood was collected from the tail vein to measure the plasma glucose level.

The glucose lowering rate was determined by the following formula.
Glucose lowering rate = [(Control group plasma glucose level - Compound-administered group plasma glucose level)/Control group plasma glucose level] x 100
The higher the glucose lowering rate of the compound, the more potent the hypoglycemic effect of the compound.

The following Compound A described as Example 26 in WO 2008/126732 was used as a comparative compound.

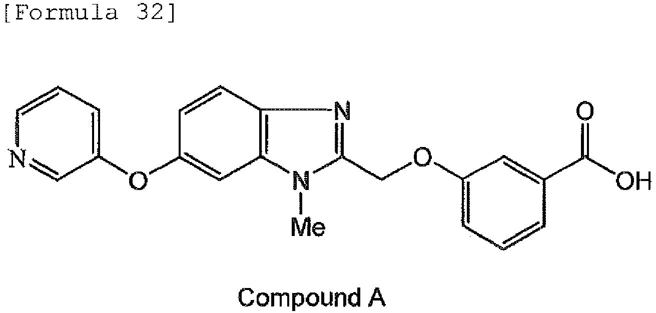

The results of comparing the compounds of Reference Examples with the comparative Compound A are shown in Table 2.

**(Table 2)**

| Reference Example | Glucose lowering rate (%) |
|---|---|
| 2 | 31 |
| 4 | 28 |
| 5 | 34 |
| 8 | 33 |
| 9 | 41 |
| 10 | 37 |
| 15 | 30 |
| Compound A | 20 |

As is clear from Table 2, the compounds of Reference Examples have a hypoglycemic effect equal to or greater than that of Compound A described in WO 2008/126732.

### (Test Example 2)

### Measurement of PPARγ activation effect/modulator activity

Rosiglitazone used in Examples is a commercially available PPARγ activator and is a compound described in U.S. Patent No. 5,002,953, and can be produced according to the method described therein.

A test was carried out according to the reporter assay method with reference to a report by Kliewer et al. (Journal of Biological Chemistry, 1995, Vol. 270 (22), p. 12953-12956) as a method for measuring the ability of a compound to activate PPARγ (hereinafter PPARγ activation effect/modulator activity). Commercially available reagents and kits were used according to the attached instructions. The details will be shown below.

### (1) Preparation of GAL4-PPARγ chimeric receptor expression plasmid

The ligand-binding domain of human PPARγ (corresponding to about 300 amino acids at the carboxy end) was bound to the DNA-binding domain of the yeast transcription factor GAL4 (corresponding to 147 amino acids at the amino end) with reference to the report by Kliewer et al. to prepare a gene expressing a GAL4-PPARγ receptor.

The base sequence of the human PPARγ gene is described in the gene database GenBank under Accession No. X90563.

### (1-1) Extraction of total RNA from cell line HepG2

The cell line HepG2 (American Type Culture Collection HB-8065) was purchased from Dainippon Pharmaceutical Co., Ltd. and cultured in a tissue culture flask having a culture area of 75 cm² (manufactured by BD Biosciences). Dulbecco's modified Eagle's medium (Gibco D-MEM, manufactured by Invitrogen Corporation) supplemented with fetal bovine serum (manufactured by HyClone) at a volume ratio of 10% and an antibiotic solution [Antibiotic Antimycotic Solution, stabilized (100 x), manufactured by Sigma] at a volume ratio of 1% was used as a medium.

The cells were cultured in a carbon dioxide incubator at 37°C under 5% carbon dioxide for three days. When the cells were grown to an approximately semiconfluent state, the medium in the flask was removed by aspiration. The cells were washed by adding 10 ml of ice-cooled phosphate-buffered saline (Gibco Dulbecco's Phosphate-Buffered Saline, manufactured by Invitrogen Corporation), and then the saline was removed by aspiration. Thereafter, 7.5 ml of Trizol reagent (Gibco TRIZOL reagent, manufactured by Invitrogen Corporation) was added to the cells in the flask, and repeatedly pipetted. The cells were lysed by incubating at room temperature for about five minutes.

The cell lysate was subjected to precipitation with isopropyl alcohol according to the instructions of the Trizol reagent. The resulting RNA precipitate was dissolved in pure water and stored in a freezer at about -20°C. Here, the volume of the RNA solution was 0.22 ml. A sample obtained by diluting a part of the RNA solution 100-fold with pure water had an absorbance at 260 nm of 0.562. The yield of the total RNA was calculated to be 0.562 × 100 × 39.5 × 0.22 = 488 µg assuming that 39.5 µg/ml of RNA was present when the absorbance was 1.

### (1-2) Cloning of cDNA of PPARγ ligand-binding domain

Two oligonucleotides represented by SEQ ID NOS: 1 and 2 in the later-described Sequence Listing, as designed based on the gene sequence of human PPARγ, were chemically synthesized as primers for amplification by reverse transcript polymerase chain reaction (hereinafter RT-PCR) of cDNA of the PPARγ ligand-binding domain using Beckman Oligo 1000 (manufactured by Beckman).

cDNA of PPARγ was amplified by RT-PCR using Ready-To-Go RT-PCR Beads (manufactured by Amersham Pharmacia Biotech, Inc.) with the HepG2 total RNA previously obtained as a template and the two oligonucleotides as primers. The reaction product was subjected to 1.5% agarose electrophoresis. The amplified band of about 900 base pairs was cut out, purified, and cloned to the plasmid pCRII (manufactured by Invitrogen Corporation). The amplified DNA fragment is assumed to have the nucleotide sequence represented by SEQ ID NO: 3 of the Sequence Listing which includes a sequence encoding the ligand-binding domain, specifically, amino acids 175 to 475, of human PPARγ, and to which a restriction enzyme BamHI cleavage site and a restriction enzyme HindIII site are added on the 5'-terminal and 3'-terminal, respectively. The plasmid clone correctly containing the sequence represented by SEQ ID NO: 3 was selected by confirming the nucleotide sequence.

### (1-3) Production of plasmid pM-PPARγ

Next, the selected plasmid was treated with restriction enzymes BamHI and HindIII to obtain a 900-base-pair fragment containing the gene of the PPARγ ligand-binding domain. This was inserted into the BamHI-HindIII site of the plasmid pM having the gene of the DNA-binding domain of the yeast transcription factor GAL4 (manufactured by Clontech Laboratories, Inc.) and cloned.

The plasmid pM-PPARγ obtained by the above operation includes the nucleotide sequence represented by SEQ ID NO: 4 of the Sequence Listing and encodes an amino acid sequence represented by SEQ ID NO: 5 of the Sequence Listing containing amino acids 1 to 147 of the yeast transcription factor GAL4 at the amino end and containing amino acids 175 to 475 of human PPARγ and a stop codon at the carboxy end. The plasmid is a gene that can express a GAL4-PPARγ chimeric receptor in mammalian cells.

### (2) Measurement of PPARγ activation ability

The previously acquired plasmid pM-PPARγ and the plasmid pFR-Luc purchased from Stratagene Cloning Systems, Inc. were dissolved in deionised water at a concentration of 1 mg/mL each.

The monkey kidney-derived cell line COS-7 (American Type Culture Collection CRL-1651) was seeded into a 75 cm² culture flask and cultured using Dulbecco's modified Eagle's medium containing 10% fetal bovine serum (hereinafter medium) under the conditions of 37°C and 5% carbon dioxide gas until an approximately 80% confluent state was obtained.

COS-7 cells were transfected with 4.8 micrograms per flask of the plasmid pM-PPARγ and 19.2 µg per flask of the plasmid pFR-Luc using Lipofectamine 2000 transfection reagent (manufactured by Invitrogen Corporation), and the cells were cultured overnight.

On the following day, the cells were harvested by trypsin treatment, suspended in 75 mL of phenol red-free Dulbecco's modified Eagle's medium containing 10% fetal bovine serum, seeded into a white 96-well plate (manufactured by Costar) using the medium in a volume of 95 µL per well, and cultured overnight.

The test compound was dissolved in dimethyl sulfoxide at a concentration of 4 mM. The solution was serially diluted 3.3-fold with dimethyl sulfoxide to prepare solutions of the compound at concentrations up to 400 nM. Dimethyl sulfoxide was prepared for the control group. Rosiglitazone dissolved in dimethyl sulfoxide at a concentration of 4 mM was prepared for the positive control group. They were diluted 20-fold with the medium, and 5 µL of the dilution was added to the wells in which the cells were grown. The concentrations of the test compound treating the cells ranged from 10 µM to 1 nM. After the addition, the cells were cultured overnight.

On the following day, the medium was removed, and Luc Lite (manufactured by PerkinElmer Inc.) was prepared according to the attached document and added at 50 microliters per well. The plate with cells in the Luc Lite was stirred for about 30 minutes. The amount of luminescence in each well was measured as luciferase activity using Analyst (Molecular Devices) for 0.5 second. A dose-dependent curve was drawn.

When the luciferase activity of the positive control group was 100% and the luciferase activity of the control group was 0%, the maximum luciferase activity exhibited by the test compound alone was calculated as Emax (%) and the concentration of the test compound represented by Emax/2 was calculated as EC50.

The smaller the EC50 value of the compound, the more potent the PPARγ activation effect/modulator activity of the compound.

Compound A used in Test Example 1 was used as a comparative compound.

The results of comparing the compounds of Reference Examples with the comparative Compound A are shown in Table 3.

**(Table 3)**

| Reference Example | EC₅₀ (nM) | Emax (%) |
|---|---|---|
| 2 | 180 | 73 |
| 4 | 180 | 81 |
| 5 | 24 | 79 |
| 8 | 100 | 100 |
| 9 | 43 | 74 |
| 10 | 51 | 60 |
| 11 | 260 | 82 |
| 12 | 71 | 83 |
| 14 | 69 | 67 |
| 15 | 120 | 94 |
| Compound A | 6800 | 66 |

As shown in Table 3, the compounds of Reference Examples have PPARγ activation effect/modulator activity equal to or greater than that of Compound A described in WO 2008/126732. Accordingly, the compounds of Reference Examples are assumed to be useful as therapeutic agents or prophylactic agents for a disease based on dyslipidemia, arteriosclerosis, hyperlipidemia, diabetes, involutional osteoporosis, adiposis, cancer, or the like.

### (Formulation Example 1)

| | |
|---|---|
| Capsules | |
| Compound of Reference Example 1 or 2 | 50 mg |
| Lactose | 128 mg |
| Corn starch | 70 mg |
| Magnesium stearate | 2 mg |
| | 250 mg |

The above-formulated powder is mixed and allowed to pass through a 60-mesh sieve. Then, the powder is put in 250 mg gelatin capsules to prepare capsules.

### (Formulation Example 2)

**Tablets**

| | |
|---|---|
| Compound of Reference Example 1 or 2 | 50 mg |
| Lactose | 126 mg |
| Corn starch | 23 mg |
| Magnesium stearate | 1 mg |
| | 200 mg |

The above-formulated powder is mixed, granulated using a corn starch paste, dried, and then tableted using a tableting machine to prepare tablets each having a weight of 200 mg. The tablets may be sugar-coated as necessary.

### Industrial Applicability

Novel pyridine derivatives shown in Reference Examples are useful as therapeutic agents or prophylactic agents for metabolic syndrome, specifically, diseases such as diabetes (especially type II diabetes), hyperglycemia, hyperlipidemia, adiposity, impaired glucose tolerance (IGT), insulin resistance, impaired fasting glucose (IFG), hypertension, fatty liver, nonalcoholic steatohepatitis (NASH), diabetic complications (such as retinopathy, nephropathy or neuropathy), arteriosclerosis, gestational diabetes mellitus (GDM) or polycystic ovary syndrome (PCOS), inflammatory disease (such as osteoarthritis, pain or inflammatory enteritis), acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease (such as systemic lupus erythematosus, chronic rheumatism, Sjogren's syndrome, systemic sclerosis, mixed connective tissue disease, Hashimoto's disease, Crohn's disease, ulcerative colitis, idiopathic Addison's disease, male sterility, Goodpasture's syndrome, rapidly progressive glomerulonephritis, myasthenia gravis, polymyositis, multiple sclerosis, autoimmune hemolytic anemia, idiopathic thrombocytopenic purpura, Behcet's disease or CREST syndrome), pancreatitis, cachexia, cancer (such as gastric cancer, lung cancer, breast cancer, colon cancer, prostate cancer, pancreatic cancer or liver cancer), leukemia, sarcoma (such as liposarcoma), osteoporosis, involutional osteoporosis, neurodegenerative disease, Alzheimer's disease, hyperuricemia, dry eyes, or the like and are expected to be used as medicines. The present invention provides a process for preparing 3-(6-hydroxy-1-methyl-1H-benzimidazol-2-yl)benzoic acid esters as synthetic intermediates for these novel pyridine derivatives with high quality, in a few steps and in a high yield.

### Sequence Listing Free Text

SEQ ID NO: 1: PCR sense primer
SEQ ID NO: 2: PCR antisense primer
SEQ ID NO: 3: Nucleotide sequence of synthetic human PPARγ cDNA
SEQ ID NO: 4: Nucleotide sequence of GAL4 chimeric PPARγ receptor gene
SEQ ID NO: 5: Amino acid sequence of GAL4 chimeric PPARγ receptor

## Claims

1. A process for preparing a compound represented by general formula (3): 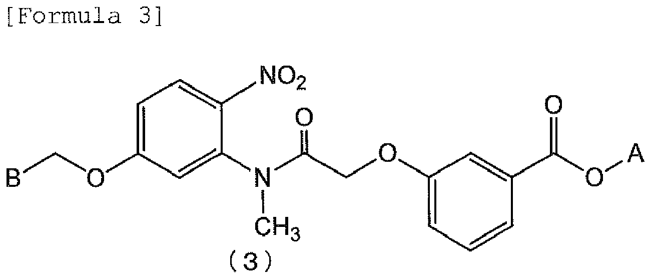 [wherein
A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group; and B represents a C₆-C₁₀ aryl group which may be substituted with a C₁-C₆ alkyl group or a C₁-C₆ alkoxy group], the process comprising reacting a compound represented by general formula (1): 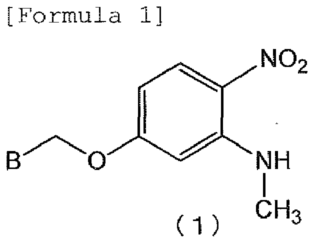 [wherein B is as defined above] with a compound represented by general formula (2): 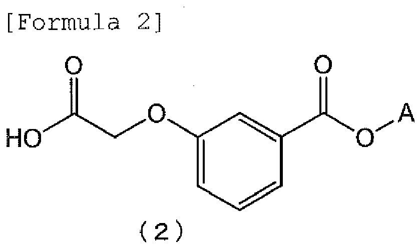 [wherein A is as defined above] in a solvent.

2. The preparation process according to claim 1, wherein A is a methyl group.

3. The preparation process according to claim 1 or 2, wherein B is a phenyl group.

4. The preparation process according to any one of claims 1 to 3, wherein the solvent is a halogenated hydrocarbon, a nitrile, an ether or a mixed solvent thereof.

5. The preparation process according to any one of claims 1 to 3, wherein the solvent is tetrahydrofuran.

6. The preparation process according to any one of claims 1 to 3, wherein the solvent is a halogenated hydrocarbon, a nitrile, an ether, an amide, a carboxylate or a mixed solvent thereof.

7. The preparation process according to any one of claims 1 to 3, wherein the solvent is N,N-dimethylacetamide.

8. The preparation process according to any one of claims 1 to 7 in the presence of a halogenating agent.

9. The preparation process according to claim 8, wherein the halogenating agent is thionyl chloride, oxalyl chloride or phosphorus pentachloride.

10. The preparation process according to claim 8, wherein the halogenating agent is thionyl chloride.

11. The preparation process according to any one of claims 8 to 10, wherein the compound represented by the general formula (1) and the compound represented by the general formula (2) are previously mixed and the halogenating agent is added thereto.

12. The preparation process according to any one of claims 1 to 10 in the presence of a base.

13. The preparation process according to claim 12, wherein the base is an alkali metal hydride.

14. The preparation process according to claim 12, wherein the base is sodium hydride.

15. The preparation process according to any one of claims 1 to 14 in the presence of a catalyst.

16. The preparation process according to claim 15, wherein the catalyst is N,N-dimethylformamide.

17. A process for preparing a compound represented by general formula (4) : 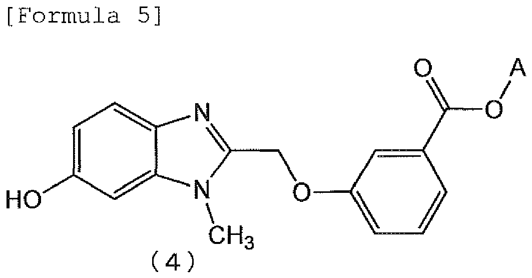 [wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group], the process comprising reacting a compound represented by general formula (3): 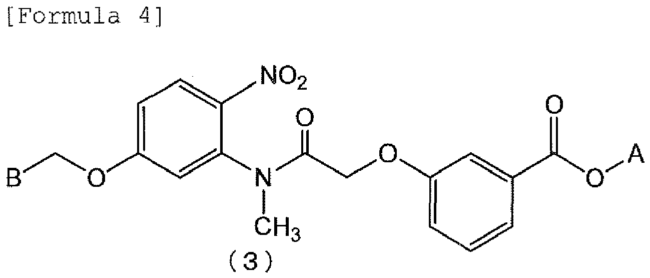 [wherein A is as defined above; and B represents a C₆-C₁₀ aryl group which may be substituted with a C₁-C₆ alkyl group or a C₁-C₆ alkoxy group] with hydrogen in a solvent in the presence of a catalyst.

18. The preparation process according to claim 17, wherein A is a methyl group.

19. The preparation process according to claim 17 or 18, wherein B is a phenyl group.

20. The preparation process according to any one of claims 17 to 19, wherein the solvent is an alcohol, an amide or a mixed solvent thereof.

21. The preparation process according to any one of claims 17 to 19, wherein the solvent is N,N-dimethylacetamide.

22. The preparation process according to any one of claims 17 to 21, wherein the catalyst is a palladium-carbon catalyst or a platinum-carbon catalyst.

23. The preparation process according to any one of claims 17 to 21, wherein the catalyst is a palladium-carbon catalyst.

24. A process for preparing a compound represented by general formula (5): 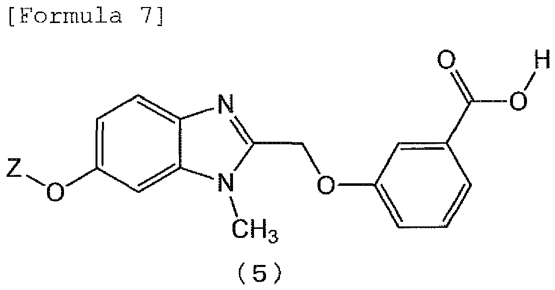 [wherein Z represents a pyridyl group independently substituted with 1 to 3 halogen atom(s), C₁-C₆ alkyl group(s) and/or C₁-C₆ alkoxy group(s)] using a compound produced in the following Step I and represented by general formula (3): 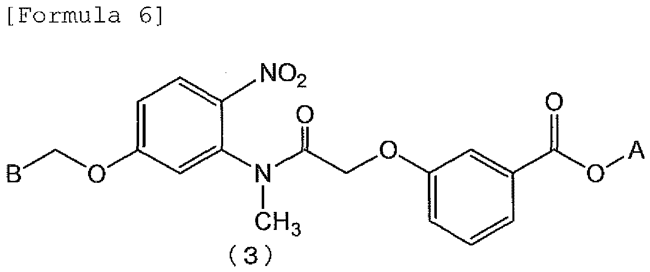 [wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group; and B represents a C₆-C₁₀ aryl group which may be substituted with a C₁-C₆ alkyl group or a C₁-C₆ alkoxy group]
{wherein Step I is a step of preparing the compound represented by the general formula (3), the step comprising reacting a compound represented by general formula (1) : 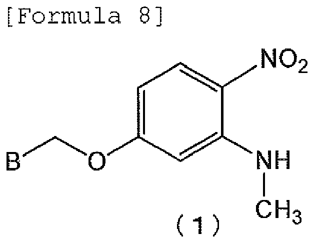 [wherein B is as defined above] with a compound represented by general formula (2): 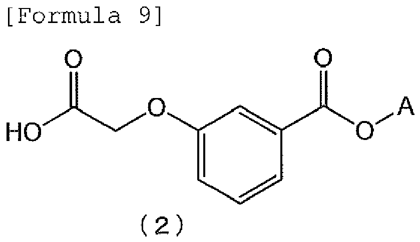 [wherein A is as defined above] in a solvent}.

25. A process for preparing a compound represented by general formula (5) : 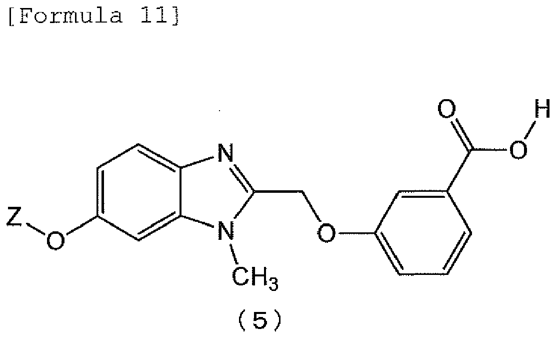 [wherein Z represents a pyridyl group independently substituted with 1 to 3 halogen atom(s), C₁-C₆ alkyl group(s) and/or C₁-C₆ alkoxy group(s)] using a compound produced in the following Step II and represented by general formula (4): 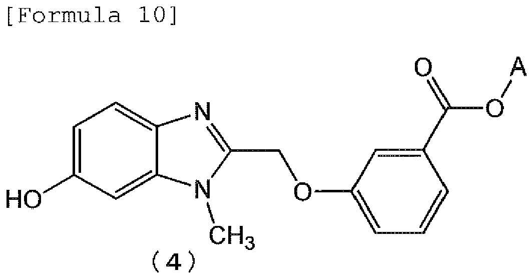 [wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group]
{wherein Step II is a step of preparing the compound represented by the general formula (4), the step comprising reacting a compound represented by general formula (3): 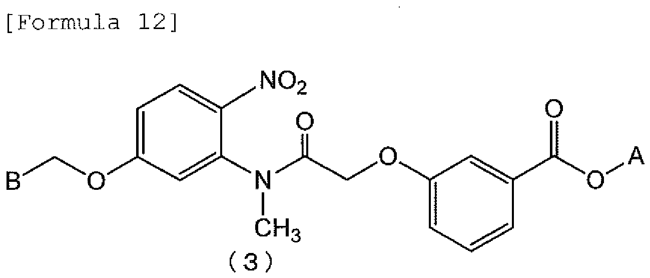 [wherein A is as defined above; and B represents a C₆-C₁₀ aryl group which may be substituted with a C₁-C₆ alkyl group or a C₁-C₆ alkoxy group] with hydrogen in a solvent in the presence of a catalyst}.

26. A compound represented by general formula (3): 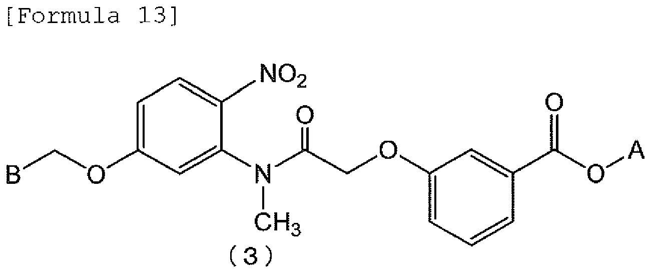 [wherein A represents a C₁-C₆ alkyl group which may be substituted with a C₁-C₆ alkoxy group; a C₃-C₇ cycloalkyl group which may be substituted with a C₁-C₆ alkyl group or a C₁-C₆ alkoxy group; or a C₆-C₁₀ aryl group which may be substituted with a C₁-C₆ alkyl group or a C₁-C₆ alkoxy group; and B represents a C₆-C₁₀ aryl group which may be substituted with a C₁-C₆ alkyl group or a C₁-C₆ alkoxy group].

27. The compound according to claim 26, wherein A is a C₁-C₆ alkyl group.

28. The compound according to claim 26, wherein A is a methyl group.

29. The compound according to any one of claims 26 to 28, wherein B is a C₆-C₁₀ aryl group.

30. The compound according to any one of claims 26 to 28, wherein B is a phenyl group.


### Sequence Listing

SEQUENCE LISTING

<110>  DAIICHI-SANKYO COMPANY, LIMITED

<120>  Method for producing benzoate

<130>  FP1137

<150>  JP2010-200092
<151>  2010-09-07

<160>  5

<170>  PatentIn version 3.3

<210>  1
<211>  29
<212>  DNA
<213>  Artificial

<220>
<223>  Inventor: Wakayama, Masakazu; Saito, Ayako
       Inventor: Kajino, Hisaki

<220>
<223>  PCR sense primer

<400>  1
ggatccataa tgccatcagg tttgggcgg                                        29

<210>  2
<211>  30
<212>  DNA
<213>  Artificial

<220>
<223>  PCR antisense primer

<400>  2
aagcttctag tacaagtcct tgtagatctc                                       30

<210>  3
<211>  917
<212>  DNA
<213>  Artificial Sequence

<220>
<223>  ScDNA coding ligand binding domain of human PPAR gamma

<400>  3
ggatccataa tgccatcagg tttgggcgga tgccacaggc cgagaaggag aagctgttgg      60

cggagatctc cagtgatatc gaccagctga atccagagtc cgctgacctc cgggccctgg     120

caaaacattt gtatgactca tacataaagt ccttcccgct gaccaaagca aaggcgaggg     180

cgatcttgac aggaaagaca acagacaaat caccattcgt tatctatgac atgaattcct     240

taatgatggg agaagataaa atcaagttca aacacatcac ccccctgcag gagcagagca     300

aagaggtggc catccgcatc tttcagggct gccagtttcg ctccgtggag gctgtgcagg     360

agatcacaga gtatgccaaa agcattcctg gttttgtaaa tcttgacttg aacgaccaag 420

taactctcct caaatatgga gtccacgaga tcatttacac aatgctggcc tccttgatga 480

ataaagatgg ggttctcata tccgagggcc aaggcttcat gacaagggag tttctaaaga 540

gcctgcgaaa gccttttggt gactttatgg agcccaagtt tgagtttgct gtgaagttca 600

atgcactgga attagatgac agcgacttgg caatatttat tgctgtcatt attctcagtg 660

gagaccgccc aggtttgctg aatgtgaagc ccattgaaga cattcaagac aacctgctac 720

aagccctgga gctccagctg aagctgaacc accctgagtc ctcacagctg tttgccaagc 780

tgctccagaa aatgacagac ctcagacaga ttgtcacgga acacgtgcag ctactgcagg 840

tgatcaagaa gacggagaca gacatgagtc ttcacccgct cctgcaggag atctacaagg 900

acttgtacta gaagctt 917

<210> 4
<211> 1365
<212> DNA
<213> Artificial

<220>
<223> GAL4-PPAR gamma chimeric receptor

<220>
<221> CDS
<222> (1)..(1365)

<400> 4
atg aag cta ctg tct tct atc gaa caa gca tgc gat att tgc cga ctt 48
Met Lys Leu Leu Ser Ser Ile Glu Gln Ala Cys Asp Ile Cys Arg Leu
1               5                   10                  15

aaa aag ctc aag tgc tcc aaa gaa aaa ccg aag tgc gcc aag tgt ctg 96
Lys Lys Leu Lys Cys Ser Lys Glu Lys Pro Lys Cys Ala Lys Cys Leu
            20                  25                  30

aag aac aac tgg gag tgt cgc tac tct ccc aaa acc aaa agg tct ccg 144
Lys Asn Asn Trp Glu Cys Arg Tyr Ser Pro Lys Thr Lys Arg Ser Pro
        35                  40                  45

ctg act agg gca cat ctg aca gaa gtg gaa tca agg cta gaa aga ctg 192
Leu Thr Arg Ala His Leu Thr Glu Val Glu Ser Arg Leu Glu Arg Leu
    50                  55                  60

gaa cag cta ttt cta ctg att ttt cct cga gaa gac ctt gac atg att 240
Glu Gln Leu Phe Leu Leu Ile Phe Pro Arg Glu Asp Leu Asp Met Ile
65                  70                  75                  80

ttg aaa atg gat tct tta cag gat ata aaa gca ttg tta aca gga tta 288
Leu Lys Met Asp Ser Leu Gln Asp Ile Lys Ala Leu Leu Thr Gly Leu
                85                  90                  95

ttt gta caa gat aat gtg aat aaa gat gcc gtc aca gat aga ttg gct 336
Phe Val Gln Asp Asn Val Asn Lys Asp Ala Val Thr Asp Arg Leu Ala
            100                 105                 110

tca gtg gag act gat atg cct cta aca ttg aga cag cat aga ata agt 384
Ser Val Glu Thr Asp Met Pro Leu Thr Leu Arg Gln His Arg Ile Ser

115                 120                 125

gcg aca tca tca tcg gaa gag agt agt aac aaa ggt caa aga cag ttg      432
Ala Thr Ser Ser Ser Glu Glu Ser Ser Asn Lys Gly Gln Arg Gln Leu
    130                 135                 140

act gta tcg ccg gaa ttc ccg ggg atc cat aat gcc atc agg ttt ggg      480
Thr Val Ser Pro Glu Phe Pro Gly Ile His Asn Ala Ile Arg Phe Gly
145                 150                 155                 160

cgg atg cca cag gcc gag aag gag aag ctg ttg gcg gag atc tcc agt      528
Arg Met Pro Gln Ala Glu Lys Glu Lys Leu Leu Ala Glu Ile Ser Ser
                165                 170                 175

gat atc gac cag ctg aat cca gag tcc gct gac ctc cgg gcc ctg gca      576
Asp Ile Asp Gln Leu Asn Pro Glu Ser Ala Asp Leu Arg Ala Leu Ala
            180                 185                 190

aaa cat ttg tat gac tca tac ata aag tcc ttc ccg ctg acc aaa gca      624
Lys His Leu Tyr Asp Ser Tyr Ile Lys Ser Phe Pro Leu Thr Lys Ala
        195                 200                 205

aag gcg agg gcg atc ttg aca gga aag aca aca gac aaa tca cca ttc      672
Lys Ala Arg Ala Ile Leu Thr Gly Lys Thr Thr Asp Lys Ser Pro Phe
    210                 215                 220

gtt atc tat gac atg aat tcc tta atg atg gga gaa gat aaa atc aag      720
Val Ile Tyr Asp Met Asn Ser Leu Met Met Gly Glu Asp Lys Ile Lys
225                 230                 235                 240

ttc aaa cac atc acc ccc ctg cag gag cag agc aaa gag gtg gcc atc      768
Phe Lys His Ile Thr Pro Leu Gln Glu Gln Ser Lys Glu Val Ala Ile
                245                 250                 255

cgc atc ttt cag ggc tgc cag ttt cgc tcc gtg gag gct gtg cag gag      816
Arg Ile Phe Gln Gly Cys Gln Phe Arg Ser Val Glu Ala Val Gln Glu
            260                 265                 270

atc aca gag tat gcc aaa agc att cct ggt ttt gta aat ctt gac ttg      864
Ile Thr Glu Tyr Ala Lys Ser Ile Pro Gly Phe Val Asn Leu Asp Leu
        275                 280                 285

aac gac caa gta act ctc ctc aaa tat gga gtc cac gag atc att tac      912
Asn Asp Gln Val Thr Leu Leu Lys Tyr Gly Val His Glu Ile Ile Tyr
    290                 295                 300

aca atg ctg gcc tcc ttg atg aat aaa gat ggg gtt ctc ata tcc gag      960
Thr Met Leu Ala Ser Leu Met Asn Lys Asp Gly Val Leu Ile Ser Glu
305                 310                 315                 320

ggc caa ggc ttc atg aca agg gag ttt cta aag agc ctg cga aag cct     1008
Gly Gln Gly Phe Met Thr Arg Glu Phe Leu Lys Ser Leu Arg Lys Pro
                325                 330                 335

ttt ggt gac ttt atg gag ccc aag ttt gag ttt gct gtg aag ttc aat     1056
Phe Gly Asp Phe Met Glu Pro Lys Phe Glu Phe Ala Val Lys Phe Asn
            340                 345                 350

gca ctg gaa tta gat gac agc gac ttg gca ata ttt att gct gtc att     1104
Ala Leu Glu Leu Asp Asp Ser Asp Leu Ala Ile Phe Ile Ala Val Ile
        355                 360                 365

att ctc agt gga gac cgc cca ggt ttg ctg aat gtg aag ccc att gaa     1152
Ile Leu Ser Gly Asp Arg Pro Gly Leu Leu Asn Val Lys Pro Ile Glu
    370                 375                 380

gac att caa gac aac ctg cta caa gcc ctg gag ctc cag ctg aag ctg      1200
Asp Ile Gln Asp Asn Leu Leu Gln Ala Leu Glu Leu Gln Leu Lys Leu
385                 390                 395                 400

aac cac cct gag tcc tca cag ctg ttt gcc aag ctg ctc cag aaa atg      1248
Asn His Pro Glu Ser Ser Gln Leu Phe Ala Lys Leu Leu Gln Lys Met
                405                 410                 415

aca gac ctc aga cag att gtc acg gaa cac gtg cag cta ctg cag gtg      1296
Thr Asp Leu Arg Gln Ile Val Thr Glu His Val Gln Leu Leu Gln Val
            420                 425                 430

atc aag aag acg gag aca gac atg agt ctt cac ccg ctc ctg cag gag      1344
Ile Lys Lys Thr Glu Thr Asp Met Ser Leu His Pro Leu Leu Gln Glu
        435                 440                 445

atc tac aag gac ttg tac tag                                          1365
Ile Tyr Lys Asp Leu Tyr
    450

<210>  5
<211>  454
<212>  PRT
<213>  Artificial

<220>
<223>  Synthetic Construct

<400>  5

Met Lys Leu Leu Ser Ser Ile Glu Gln Ala Cys Asp Ile Cys Arg Leu
1               5                   10                  15

Lys Lys Leu Lys Cys Ser Lys Glu Lys Pro Lys Cys Ala Lys Cys Leu
            20                  25                  30

Lys Asn Asn Trp Glu Cys Arg Tyr Ser Pro Lys Thr Lys Arg Ser Pro
        35                  40                  45

Leu Thr Arg Ala His Leu Thr Glu Val Glu Ser Arg Leu Glu Arg Leu
    50                  55                  60

Glu Gln Leu Phe Leu Leu Ile Phe Pro Arg Glu Asp Leu Asp Met Ile
65                  70                  75                  80

Leu Lys Met Asp Ser Leu Gln Asp Ile Lys Ala Leu Leu Thr Gly Leu
                85                  90                  95

Phe Val Gln Asp Asn Val Asn Lys Asp Ala Val Thr Asp Arg Leu Ala
            100                 105                 110

Ser Val Glu Thr Asp Met Pro Leu Thr Leu Arg Gln His Arg Ile Ser
        115                 120                 125

Ala Thr Ser Ser Ser Glu Glu Ser Ser Asn Lys Gly Gln Arg Gln Leu

130                 135                 140

Thr Val Ser Pro Glu Phe Pro Gly Ile His Asn Ala Ile Arg Phe Gly
145                 150                 155                 160

Arg Met Pro Gln Ala Glu Lys Glu Lys Leu Leu Ala Glu Ile Ser Ser
                165                 170                 175

Asp Ile Asp Gln Leu Asn Pro Glu Ser Ala Asp Leu Arg Ala Leu Ala
            180                 185                 190

Lys His Leu Tyr Asp Ser Tyr Ile Lys Ser Phe Pro Leu Thr Lys Ala
        195                 200                 205

Lys Ala Arg Ala Ile Leu Thr Gly Lys Thr Thr Asp Lys Ser Pro Phe
    210                 215                 220

Val Ile Tyr Asp Met Asn Ser Leu Met Met Gly Glu Asp Lys Ile Lys
225                 230                 235                 240

Phe Lys His Ile Thr Pro Leu Gln Glu Gln Ser Lys Glu Val Ala Ile
                245                 250                 255

Arg Ile Phe Gln Gly Cys Gln Phe Arg Ser Val Glu Ala Val Gln Glu
            260                 265                 270

Ile Thr Glu Tyr Ala Lys Ser Ile Pro Gly Phe Val Asn Leu Asp Leu
        275                 280                 285

Asn Asp Gln Val Thr Leu Leu Lys Tyr Gly Val His Glu Ile Ile Tyr
    290                 295                 300

Thr Met Leu Ala Ser Leu Met Asn Lys Asp Gly Val Leu Ile Ser Glu
305                 310                 315                 320

Gly Gln Gly Phe Met Thr Arg Glu Phe Leu Lys Ser Leu Arg Lys Pro
                325                 330                 335

Phe Gly Asp Phe Met Glu Pro Lys Phe Glu Phe Ala Val Lys Phe Asn
            340                 345                 350

Ala Leu Glu Leu Asp Asp Ser Asp Leu Ala Ile Phe Ile Ala Val Ile
        355                 360                 365

Ile Leu Ser Gly Asp Arg Pro Gly Leu Leu Asn Val Lys Pro Ile Glu
    370                 375                 380

Asp Ile Gln Asp Asn Leu Leu Gln Ala Leu Glu Leu Gln Leu Lys Leu
385                 390                 395                 400

Asn His Pro Glu Ser Ser Gln Leu Phe Ala Lys Leu Leu Gln Lys Met
                405                 410                 415

Thr Asp Leu Arg Gln Ile Val Thr Glu His Val Gln Leu Leu Gln Val
            420                 425                 430

Ile Lys Lys Thr Glu Thr Asp Met Ser Leu His Pro Leu Leu Gln Glu
        435                 440                 445

Ile Tyr Lys Asp Leu Tyr
    450